(19) Europäisches Patentamt
European Patent Office
Office européen des brevets

(11) Publication number: **0 442 448 A2**

(12) # EUROPEAN PATENT APPLICATION

(21) Application number: **91101958.6**

(22) Date of filing: **12.02.91**

(51) Int. Cl.5: **C07D 207/327**, C07D 237/14, C07D 253/07, C07D 239/36, C07D 233/72, C07D 257/04, C07D 277/30, C07D 233/64, C07D 231/12, C07D 233/84, A61K 31/40, //A61K31/41, A61K31/415,A61K31/425, A61K31/50

(30) Priority: **13.02.90 US 479507**
**13.02.90 US 479508**
**13.02.90 US 479563**
**13.02.90 US 479561**
**13.02.90 US 479564**
**13.02.90 US 479559**
**13.02.90 US 479560**
**13.02.90 US 479506**
**20.06.90 US 540988**
**13.02.90 US 479505**

(43) Date of publication of application:
**21.08.91 Bulletin 91/34**

(84) Designated Contracting States:
**AT BE CH DE DK ES FR GB GR IT LI LU NL SE**

(71) Applicant: **Bristol-Myers Squibb Company**
**345 Park Avenue**
**New York, N.Y. 10154(US)**

(72) Inventor: **Meanwell, Nicholas**
**2 Spice Hill Drive**
**East Hampton, Connecticut 06424(US)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

(54) **Heterocyclic carboxylic acids and esters.**

(57) Heterocyclic acids and esters useful as inhibitors of mammalian blood platelet aggregation characterized by Formula I, II and VIII are disclosed.

EP 0 442 448 A2

$$HET_1-(CH_2)_nCO_2R \quad (I)$$

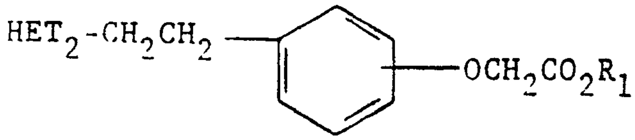

(II)

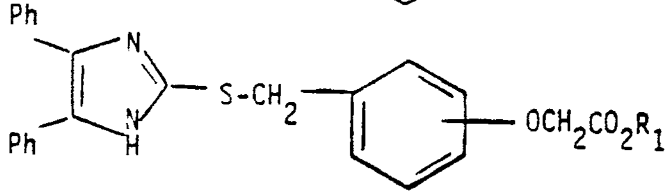

(VIII)

Formula I compounds are those wherein n is 6-9, R is hydrogen, lower alkyl or an alkali metal ion, and $HET_1$ is heterocyclic radical selected from the group consisting of 3,4-diphenyl-1H-pyrrol-1-yl, 1,6-dihydro-6-oxo-3,4-diphenyl-1-pyridazinyl, 2,3-dihydro-3-oxo-5,6-diphenyl-1,2,4-triazin-2-yl, 1-oxo-4,5-diphenyl-2H-pyrimidin-1-yl, 2,5-dioxo-3,4-diphenylimidazolidin-1-yl, 5-(diphenylmethyl)-2H-tetrazol-2-yl, 4,5-diphenyl-2-thiazoyl, 4,5-diphenyl-1H-imidazol-2-yl, and 1,5-diphenyl-1H-pyrazol-3-yl.

Formula II compounds are those wherein $R_1$ is hydrogen, lower alkyl or an alkali metal ion, and the radical $-OCH_2CO_2R$ is attached in the 3 or 4 ring position; and $HET_2$ is a heterocyclic radical selected from the group consisting of 4,5-diphenyl-2-thiazoyl, 4,5-diphenyl-1H-imidazol-2-yl,3,4-diphenyl-1H-pyrazol-1-yl, 4,5-diphenyl-1H-pyrazol-1-yl, 1-5-diphenyl-1H-pyrazol-3-yl, 1,6-dihydro-6-oxo-3,4-diphenyl-1-pyridazinyl, 2,3-dihydro-3-oxo-5,6-diphenylmethyl-1,2,3-triazin-2-yl, and 5-(diphenylmethyl)-2H-tetrazol-2-yl.

Formula VIII compounds are those wherein $R_1$ is hydrogen, lower alkyl or an alkali metal ion, and the radical $-OCH_2CO_2R$ is attached in the 3 or 4 ring position.

## HETEROCYCLIC CARBOXYLIC ACIDS AND ESTERS

### BACKGROUND OF THE INVENTION

This invention generally pertains to heterocyclic carbon compounds having drug and bio-affecting properties and to their preparation and use. In particular, the invention is concerned with various heterocyclic derivatives characterized by Formulas I and II, infra., which are inhibitors of blood platelet aggregation.

United States patents relating to the carboxylic acid and ester derivatives of the various heterocycles disclosed herein are as follows:

(1) Pyrrole compounds in Field of Search 514/427, 428, and 548/562, 563.

Chinn, U.S. Patent 3,471,513 discloses 2-(2-carboxyethyl)-5-phenyl-1-pyrrolebutyric acid and congeners with antibiotic and ulcer-inhibiting properties of formula (1)

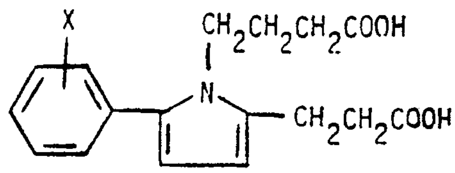

(1)

Ohsumi, et al., U.S. Patent 4,336,194 discloses carboxylic acid esters having insecticidal properties of formula (2).

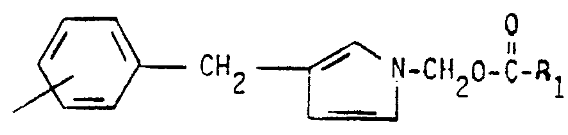

(2)

Maldonado, et al., U.S. Patent 4,563,477 discloses alpha-(N-pyrrolyl)-derivative acids having platelet antiaggregation and analgesic acvitiy of formula (3).

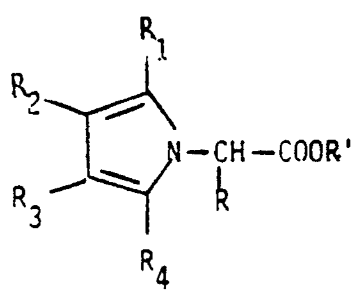

(3)

Chinn, U.S. Patent 4,694,018 discloses substituted 1,5-diphenyl-2-pyrrolepropionic acids having 5-lipoxygenase inhibiting properties of formula (4).

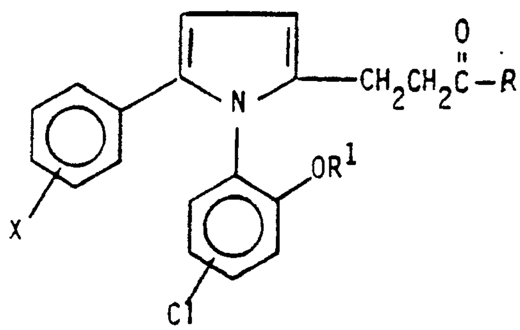

(4)

(2) Pyridazine compounds in Field of Search 514/247 and 544/239.

King, et al. U.S. Patent 2,712,542 discloses 2-(3-pyridazonyl)-acids having analgesic, anticonvulsant, antispasmodic and sedative activities of formula (5).

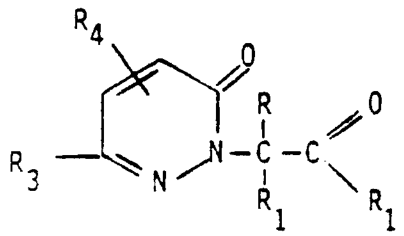

(5)

Baetz, U.S. Patent 3,622,576 discloses diphenyl-pyridazones of formula (6) having psychotropic and analgesic properties.

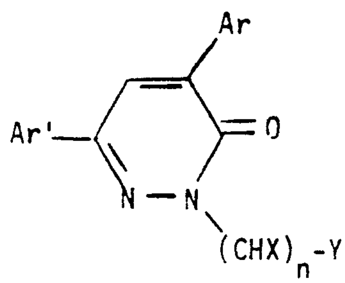

(6)

Houlihan, U.S. Patent 4,162,317 discloses succinic acid esters of 4,5-dihydro-3-(2H)-pyridizones having muscle relaxant activity of formula (7).

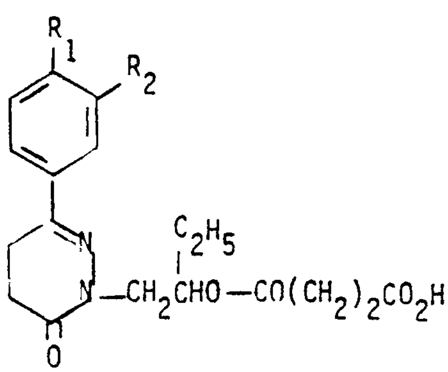

(7)

(3) Triazine compounds in Field of Search 514/242 and 544/182.

Pitet, et al., U.S. Patent 4,167,566 discloses 2-prenyl-3-oxo-5,6-diaryl-AS-triazines having antithrombogenic properties of formula (8).

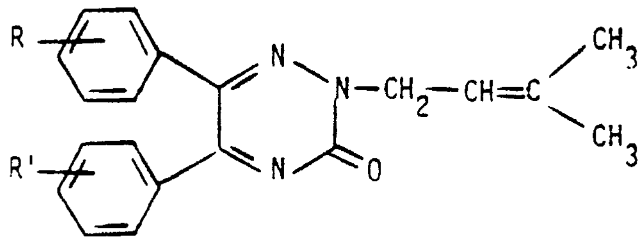

(8)

Lacefield, U.S. Patent 4,318,911 discloses 5,6-diaryl-1,2,4-triazines having antithrombogenic activity of the formula (9).

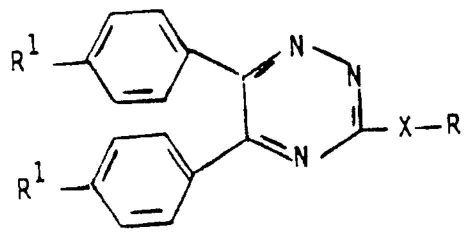

(9)

Pitet, et al., U.S. Patent 4,511,566 discloses 2-N-cycloalkylmethyl-3-oxo-5,6-diaryl-as-triazines having analgesic activity of formula (10).

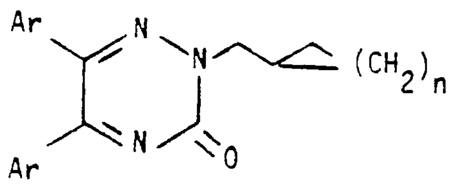

(10)

Guv, et al., U.S. Patent 4,677,105 discloses 2-alkoxycarbonylalkyl-3-oxo-5,6-diaryl-as-triazines having non-inflammatory analgesic activity of formula (11).

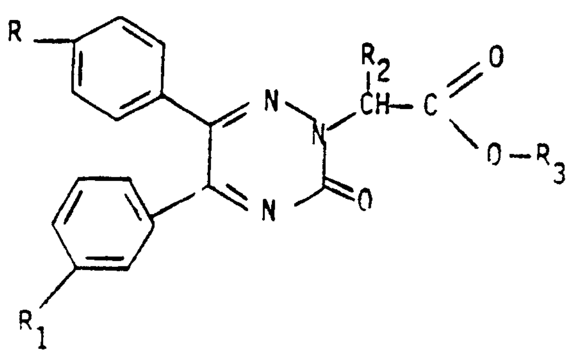

(11)

Pitit, et al., U.S. Patent 4,851,411 discloses 5-monoaryl as-triazin-3-ones having antianxiety or antidepressant activity of formula (12).

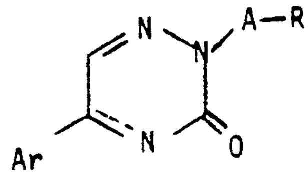

(12)

(4) Pyrimidine compounds in Field of Search 514/274 and 544/335.

Caldwell, et al., U.S. Patent 4,486,443 discloses hydantoins having antithrombogenic properties of formula (13).

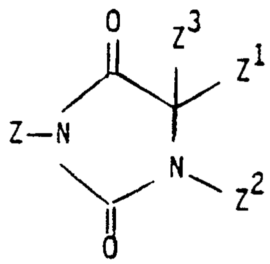

(13)

(5) Imidazolidine compounds in Field of Search 514/398 and 548/308, 312, 314.

van der Burg, U.S. Patent 3,965,114 discloses imidazolidine derivatives having anticholinergic and anticonvulsant properties of formula (14).

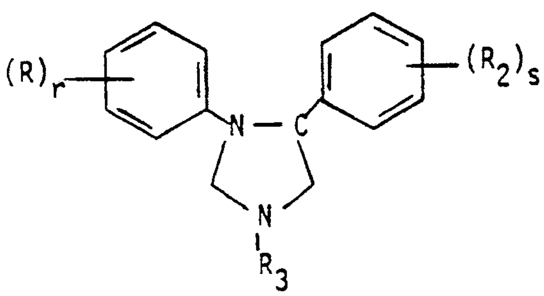

(14)

Whootton, et al., U.S. Patent 4,237,131 discloses hydantoins having bronchodilator activity of formula (15).

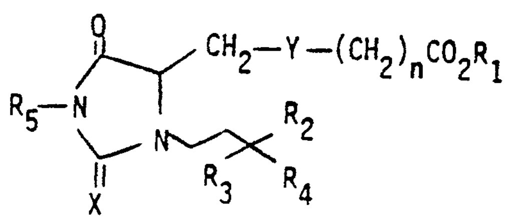

(15)

Lautenschlager, et al., U.S. Patent 4,330,550 discloses omega-(2-oxo-4-imidazolin-1-yl)alkanoic acids and esters having antithrombogenic and analgesic activity of formula (16).

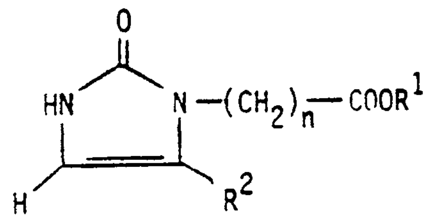

(16)

Mai, et al., U.S. Patent 4,709,042 discloses a process for preparing 2-(1-hydroxyalkyl)-5,5-diphenyl hydantoin of the formula (17).

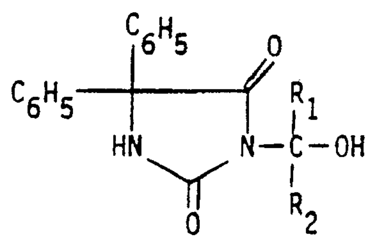

(17)

Matsuno,et al., U.S. Patent 4,797,493 discloses imidazolines having ultraviolet-absorbing properties of formula (18).

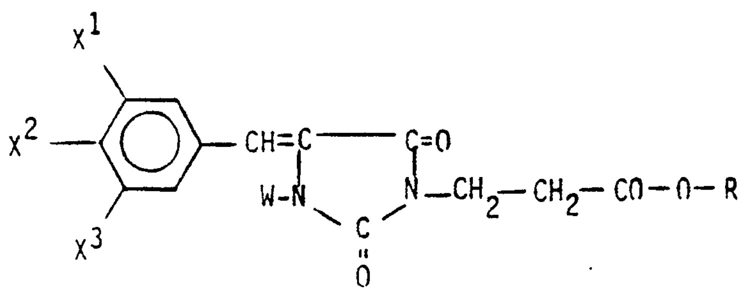

(18)

(6) Tetrazole compounds in Field of Search 514/365 and 548/253.

Hayao, U.S. Patent 3,453,285 discloses tetrazolyl alkanoic acids having anti-inflammatory properties of formula (19).

(19)

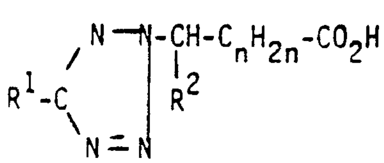

Katner, U.S. Patent 3,962,272 discloses 1H-tetrazole-1-acetate esters and acids useful for preparing cephalosporin and penicillin antibiotics of formula (20)

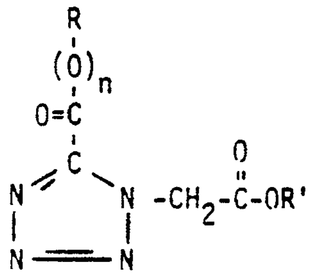

(20)

Burrell, et al., U.S. Patent 4,059,703 discloses tetrazole compounds having pesticidal activity of formula (21).

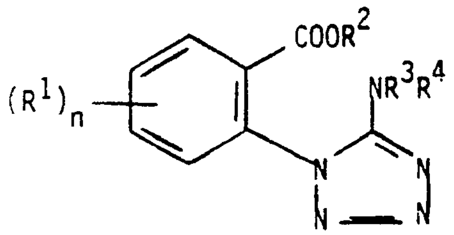

(21)

Bison, et al., U.S. Patent 4,652,671 discloses a process for the preparation of dichloroisonitrilocarboxylic acid esters of formula (22).

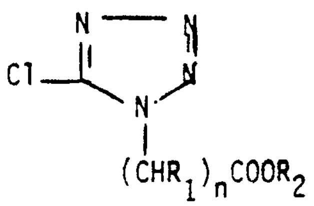

(22)

(7) Thiazole compounds in Field of Search 514/365 and 548/203, 204.
Cavalla, et al., U.S. Patent 3,506,679 discloses 2,5- and 4,5-diarylthiazolyl lower fatty acids having anti-inflammatory activity of formula (23) wherein $R^3$ is a straight chain or branched aliphatic acid radical containing 2-6 carbon atoms.

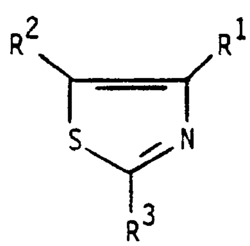

(23)

Hepworth, et al., U.S. Patent 3,538,107 discloses aryl-thiazolyl-acetic acid derivatives having anti-inflammatory, analgesic and antipyretic properties of formula (24) such as alpha-[2-(4-chlorophenyl)-thiazol-4-yl]propionic acid.

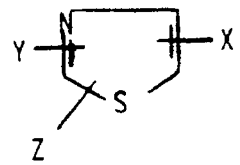

(24)

Hepworth, et al., U.S. Patent 3,661,920 discloses thiazole derivatives having anti-inflammatory, analgesic and antipyretic activity of formula (25).

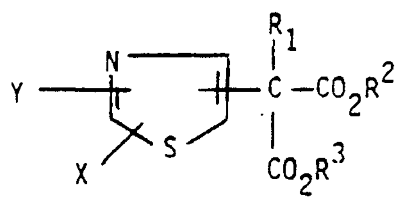

(25)

Malen, et al., U.S. Patent 3,821,237 discloses thiazolyl benzoic acid compounds having fibrinolytic, platelet stickiness decreasing and antiulcer properties of formula (26).

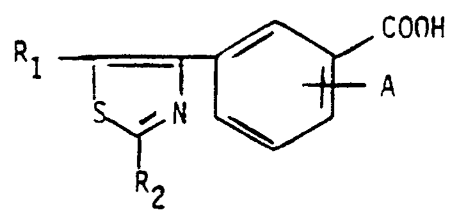

(26)

Malen, et al., U.S. Patent 3,840,548 discloses thiazolylbenzoic acid compounds having thrombolytic properties of formula (27).

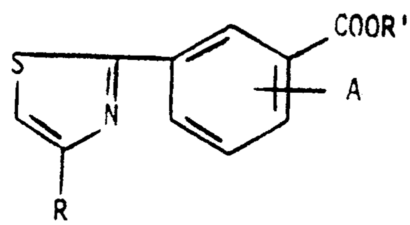

(27)

Rynbrandt, et al., U.S. Patent 4,168,315 discloses dianisyl thiazole compounds having reduced platelet adhesiveness and inhibition of platelet aggregation properties of formula (28).

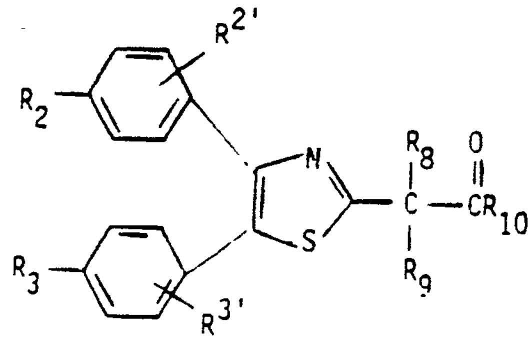

(28)

Yoshino, et al., U.S. Patent 4,659,726 discloses 4,5-bis(4-methoxyphenyl)-2-(pyrrol-2-yl)thiazoles having platelet aggregation inhibiting properties of formula (29).

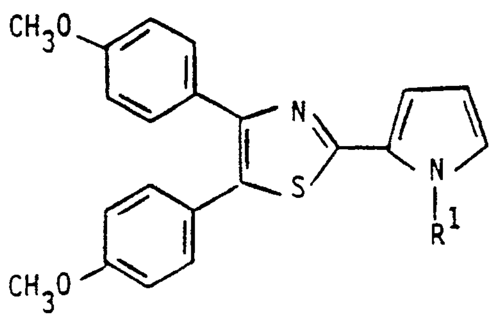

(29)

Lutomski, et al., U.S. Patent 4,791,124 discloses oxazole and thiazole compounds having insecticidal properties of formula (30).

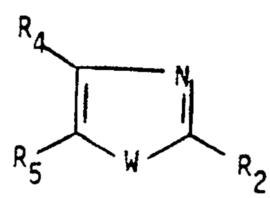

(30)

(8) Imidazole compounds in Field of Search 514/396 and 548/342.

Furukawa, et al., U.S. Patent 4,355,040 discloses imidazole-5-acetic acid derivatives havin hypotensive properties of formula (31).

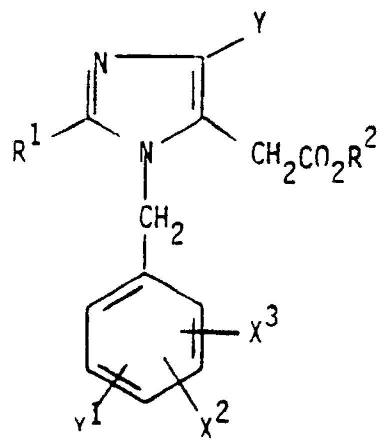

(31)

Lautenschlager, et al., U.S. Patent 4,460,598 discloses triphenylimidazolyloxyalkanoic acids having antithrombogenic properties of formula (32).

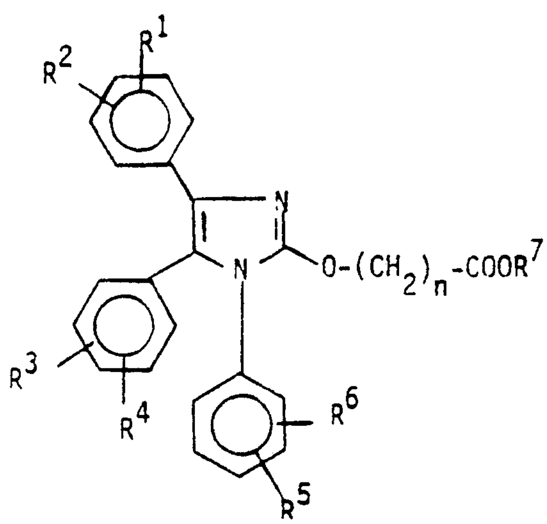

(32)

Wareing, U.S. Patent 4,668,794 discloses imidazole acrolein analogs having blood cholesterol lowering properties of formula (33).

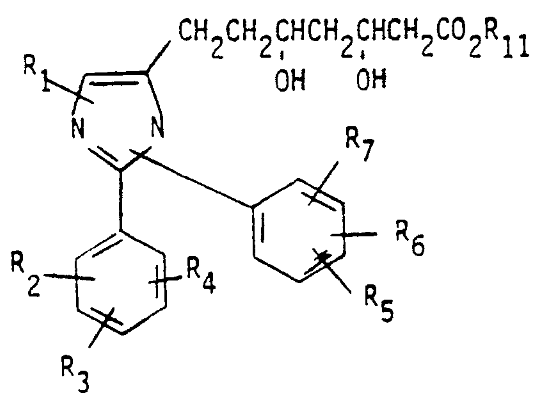

(33)

Bender, et al., U.S. Patent 4,686,231 discloses 4,5-diaryl-2(substituted)-imidazoles having 5-lipoxygenase inhibiting properties of formula (34).

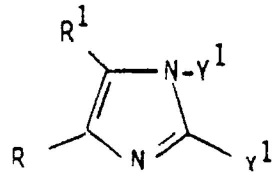

(34)

(9) Pyrazole compounds in Field of Search 514/406 and 548/378.

Noguchi, et al., U.S. Patent 3,704,241 discloses 4-pyrazole acetic acid or esters having anti-inflammatory and analgesic properties of formula (35).

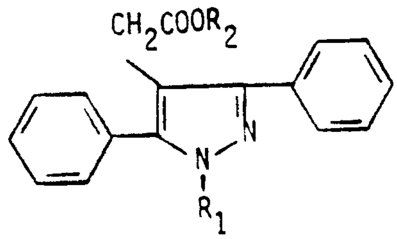

(35)

Ahrens, et al., U.S. Patent 4,042,706 discloses pyrazole derivatives having anti-inflammatory properties of formula (36).

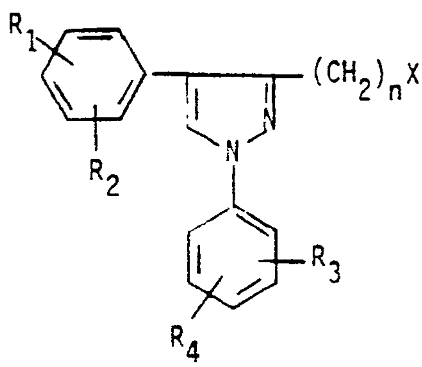

(36)

Rainer, U.S. Patent 4,146,721 discloses pyrazol-4-acetic acids having analgesic, anti-inflammatory and antipuretic properties of formula (37).

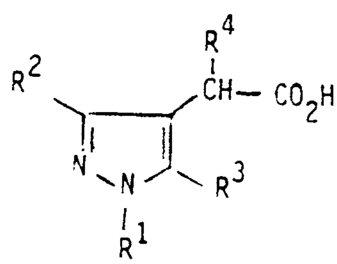

(37)

Beck, et al., U.S. Patent 4,495,195 discloses 3(5)-phenyl-substituted-5(3)-pyrazole-carboxylic acids having xanthine oxidase inhibiting peroperties of formula (38).

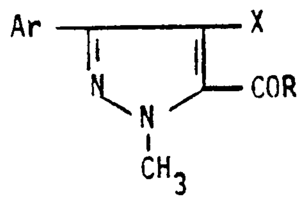

(38)

Wareing, U.S. Patent 4,613,610 discloses pyrazole analogs having cholesterol biosynthesis inhibiting properties of mevalonolactone of formula (39).

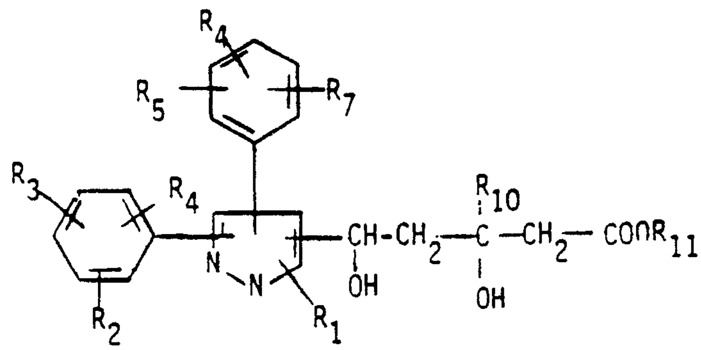

(39)

Wachter, et al., U.S. Patent 4,826,868 discloses 1,5-diaryl-3-substituted pyrazoles which inhibit cyclooxygenase enzyme and lipoxygenase enzyme pathways of formula (40).

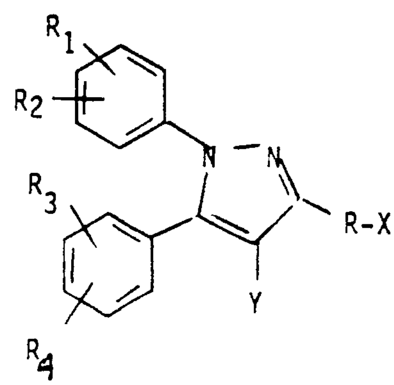

(40)

Takamura, et al., U.S. Patent 4,853,404 discloses 5-pyrazolylphenoxy acetic acids having diuretic, saluretic, and uricosuric properties of formula (41).

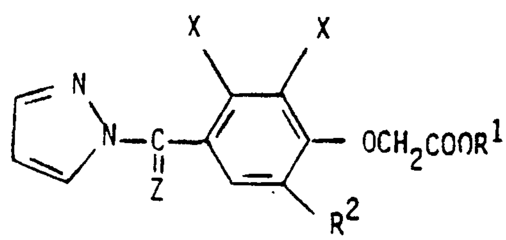

(41)

Bailey, U.S. Patent 4,870,095 discloses antiarrhythmic 1H-pyrazole-1-alkanamides of formula (42).

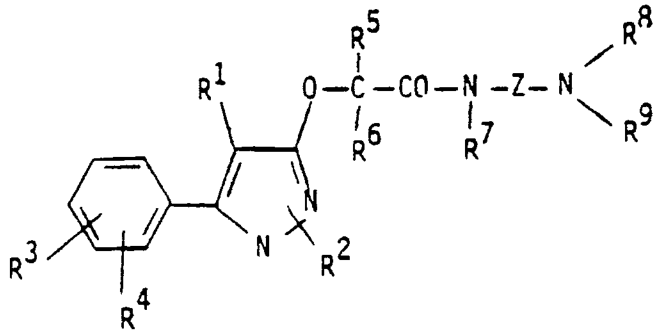

(42)

SUMMARY OF THE INVENTION

In its broadest aspect, this invention is concerned with heterocyclic carboxylic acids and esters of Formula I and Formula II

$$HET_1-(CH_2)_nCO_2R \qquad (I)$$

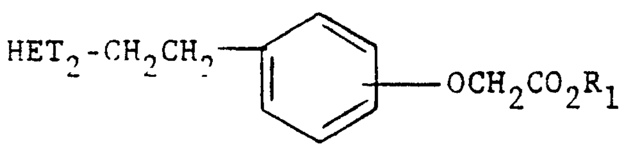

(II)

wherein $Het_1$, $Het_2$, R, $R_1$, and n are defined below which are inhibitors of adenosine diphosphate and collagen-induced aggregation of human platelet-rich plasma and are particularly useful as inhibitors of mammalian blood platelet aggregation.

Another embodiment of the invention relates to the alkali metal salts of carboxylic acids of Formula I (R is hydrogen) and Formula II ($R_1$ is hydrogen). A further embodiment concerns pharmaceutical compositions comprised of a Formula I or II compound combined with at least one pharmaceutically acceptable excipient. Yet another embodiment relates to a method for inhibiting blood platelet aggregation in a mammal which comprises administering a therapeutically effective amount of a compound of Formula I or an alkali metal salt thereof where R is hydrogen or a Formula II compound or an alkali metal salt thereof where $R_2$ is hydrogen to a mammal in need of such treatment.

DETAILED DESCRIPTION OF THE INVENTION

This invention relates to inhibitors of mammalian blood platelet aggregation OF Formula I

$HET_1-(CH_2)_nCO_2R$ (I)

wherein n is 6-9, R is hydrogen, lower alkyl or an alkali metal ion, and $HET_1$ is a heterocyclic radical selected from the group consisting of

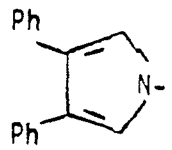

(a) 3,4-diphenyl-1H-pyrrol-1-yl,

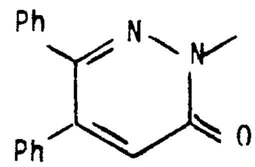

(b) 1,6-dihydro-6-oxo-3,4-diphenyl-1-pyridazinyl,

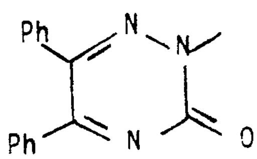

(c) 2,3-dihydro-3-oxo-5,6-diphenyl-1,2,4-triazin-2-yl,

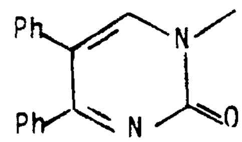

(d) 1-oxo-4,5-diphenyl-2H-pyrimidin-1-yl,

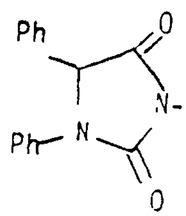

(e) 2,5-dioxo-3,4-diphenylimidazolidin-1-yl,

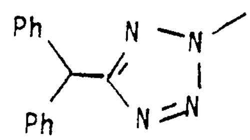

(f) 5-(diphenylmethyl)-2H-tetrazol-2-yl,

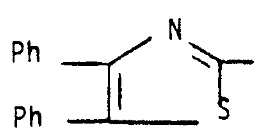

(g) 4,5-diphenyl-2-thiazoyl,

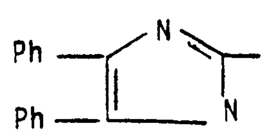

(h) 4,5-diphenyl-1H-imidazol-2-yl, and

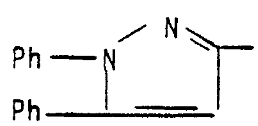

(i) 1,5-diphenyl-1H-pyrazol-3-yl.

The compounds of the instant invention are further characterized by Formula II

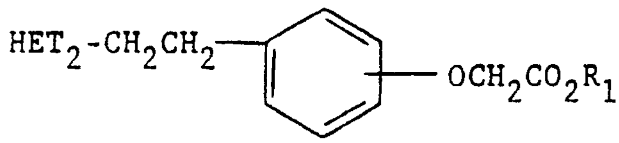

(II)

wherein

$R_1$ is hydrogen, lower alkyl or an alkali metal ion, and the radical $-OCH_7CO_2R$ is attached in the 3 or 4 ring position;

$HET_2$ is a heterocyclic radical selected from the group consisting of

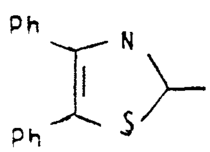

(a) 4,5-diphenyl-2-thiazoyl,

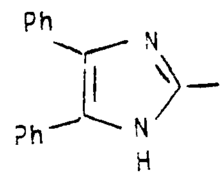

(b) 4,5-diphenyl-1H-imidazol-2-yl,

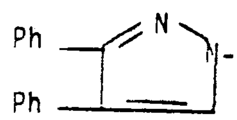

(c) 3,4-diphenyl-1H-pyrazol-1-yl,

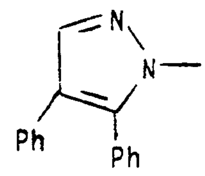

(d) 4,5-diphenyl-1H-pyrazol-1-yl,

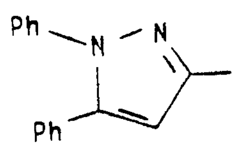

(e) 1,5-diphenyl-1H-pyrazol-3-yl,

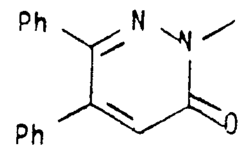

(f) 1,6-dihydro-6-oxo-3,4-diphenyl-1-pyridazinyl,

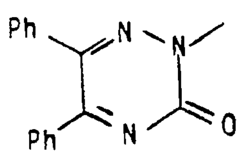

(g) 2,3-dihydro-3-oxo-5,6-diphenyl-1,2,4-triazin-2-yl, and

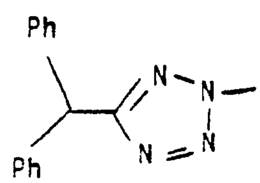

(h) 5-(diphenylmethyl)-2H-tetrazol-2-yl.

It is understood that as used herein limitations of Formula I and II are defined as follows.

The term "lower alkyl" refers to a branched or unbranched saturated hydrocarbon chain containing from 1-4 carbon atoms; specifically, methyl, ethyl, n-propyl, isopropyl, n-butyl, secondary butyl, and tertiary butyl.

The term "lower alkanol" denotes an alcohol having 1-4 carbon atoms defined by "lower alkyl".

The symbol "Ph" represents phenyl.

The term "alkali metal ion" refers to ions derived from the alkali metals, most preferably sodium and potassium.

According to the present invention the compounds characterized by Formula I are obtained by a process comprising:

(a) hydrolyzing a compound of Formula ($I^a$)

$HET_1$-$(CH_2)_nCO_2R^a$ ($I^a$)

wherein $HET_1$ is as defined above, n is 6-9 and $R^a$ is lower alkyl, or

(b) esterifying a compound of Formula ($I^b$)

$HET_1$-$(CH_2)_nCO_2H$ ($I^b$)

wherein $HET_1$ is as defined above and n is 6-9 with a lower alkanol, or

(c) alkylating $HET_1$-H wherein $HET^1$ is as defined above with a compound of Formula (III)

$X\text{-}(CH_2)_nCO_2R^a$ (III)

wherein X is halogen, preferably bromine,, n is 6 to 9, and $R^a$ is lower alkyl.

Scheme 1 below illustrates the foregoing process.

Scheme 1

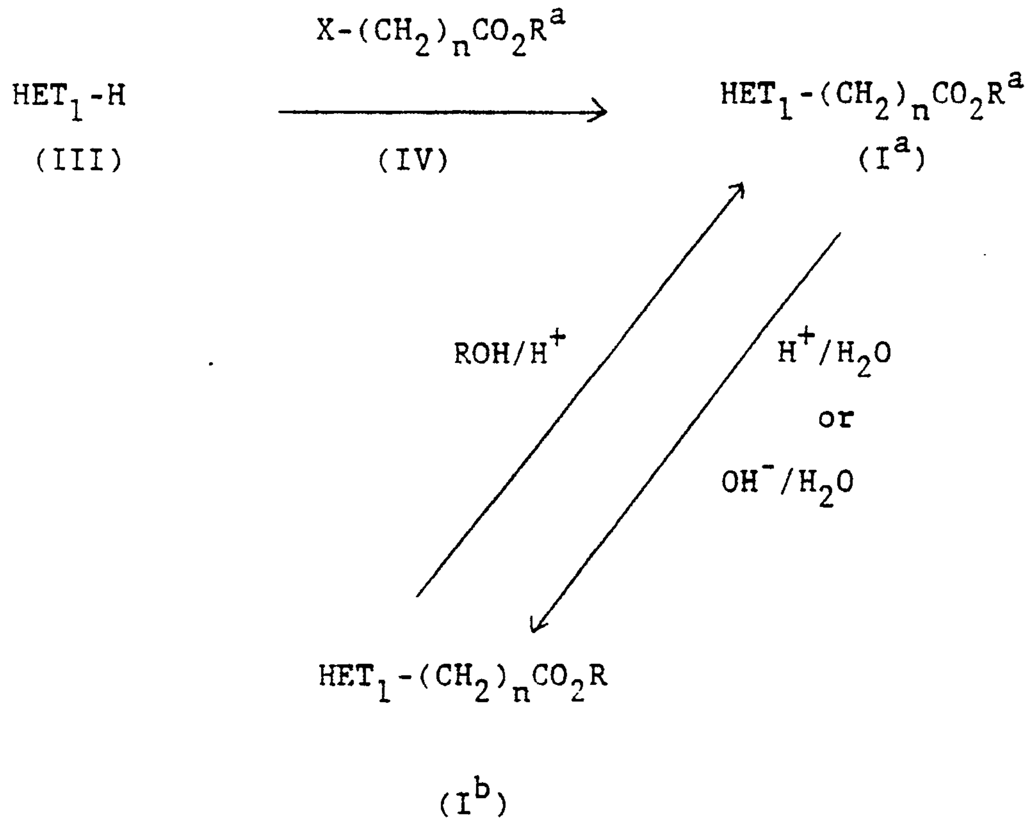


(III) (IV) ($I^a$) ($I^b$)

Compounds of Formula I are conventionally prepared as shown in Scheme 1 by base-mediated alkylation of the parent heterocycle (III) with a lower alkanol ester of an omega-halogenalkanoic acid (IV) to provide esters ($I^a$).Alkylation can be carried out with sodium hydride, potassium hydride, potassium t-butoxide in suitable solvents such as tetrahydrofuran and dimethylformamide with conditions dependent upon the identity of the heterocycle and the acidity of the proton being replaced. Preferred alkylating conditions employ sodium hydride in dimethylformamide (DMF) at room temperature or potassium carbonate in DMF at 110°C. In those instances where the alkylation results in mixtures of regioisomers, separation is readily accomplished by chromatography on silica gel. Structural assignments are made from consideration of the $^1H$ and $^{13}C$ NMR spectra. The heterocycles (III) employed as starting material are synthesized by methods reported in the chemical literature or by minor modifications thereof readily apparent to a skilled chemist.

Esters ($I^a$) are converted to the corresponding acids ($I^b$) under the influence of either aqueous alkali or aqueous acid depending upon the sensitivity of the heterocycle. Conversely, acids ($I^b$) are conventionally converted to the corresponding esters ($I^a$) by heating the acid in a lower alkanol in the presence of an inorganic acid such as hydrochloric, sulfuric and the like.

Alkali metal salts of Formula I carboxylic acids (R is an alkali metal ion) are conventionally prepared by dissolving the acid in a suitable solvent such as methanol, adding a molar equivalent of an alkali base such as sodium methoxide, and precipitating the salt or removing the solvent.

According to the present invention, the compounds characterized by Formula II are obtained by a process comprising:

(a) hydrolyzing a compound of Formula $II^a$

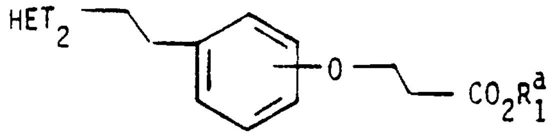

(IIᵃ)

wherein $Het_2$ is as defined above and $R_1^a$ is lower alkyl; or
(b) esterifying a compound of Formula $II^b$

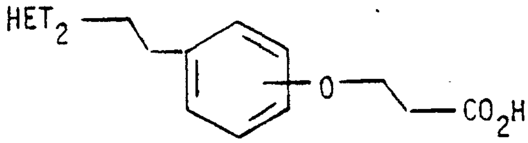

($II^b$)

wherein $HET_2$ is as defined above; or
(c) alkylating $HET_2$-H wherein $HET_2$ is as defined above with a compound of Formula (V)

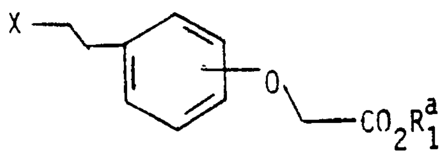

(V)

wherein $R_1^a$ is lower alkyl and X is halogen or an aryl sulfonate; or
(d) alkylating a compound of Formula (VI)

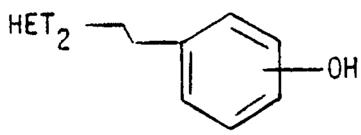

(VI)

wherein $HET_2$ is as defined above with $BrCH_2CO_2R_1^a$ wherein $R_1^a$ is lower alkyl; or
(e) hydrolyzing a compound of Formula VII

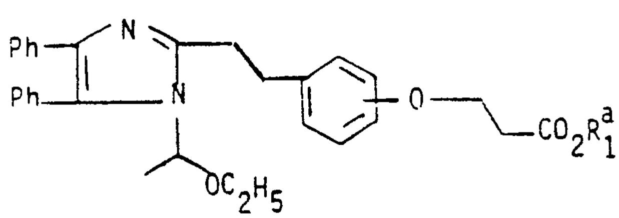

(VII)

wherein $R_1^a$ is as defined above.

The following schemes for preparation of representative compounds of Formula II illustrate the foregoing process.

Scheme 2

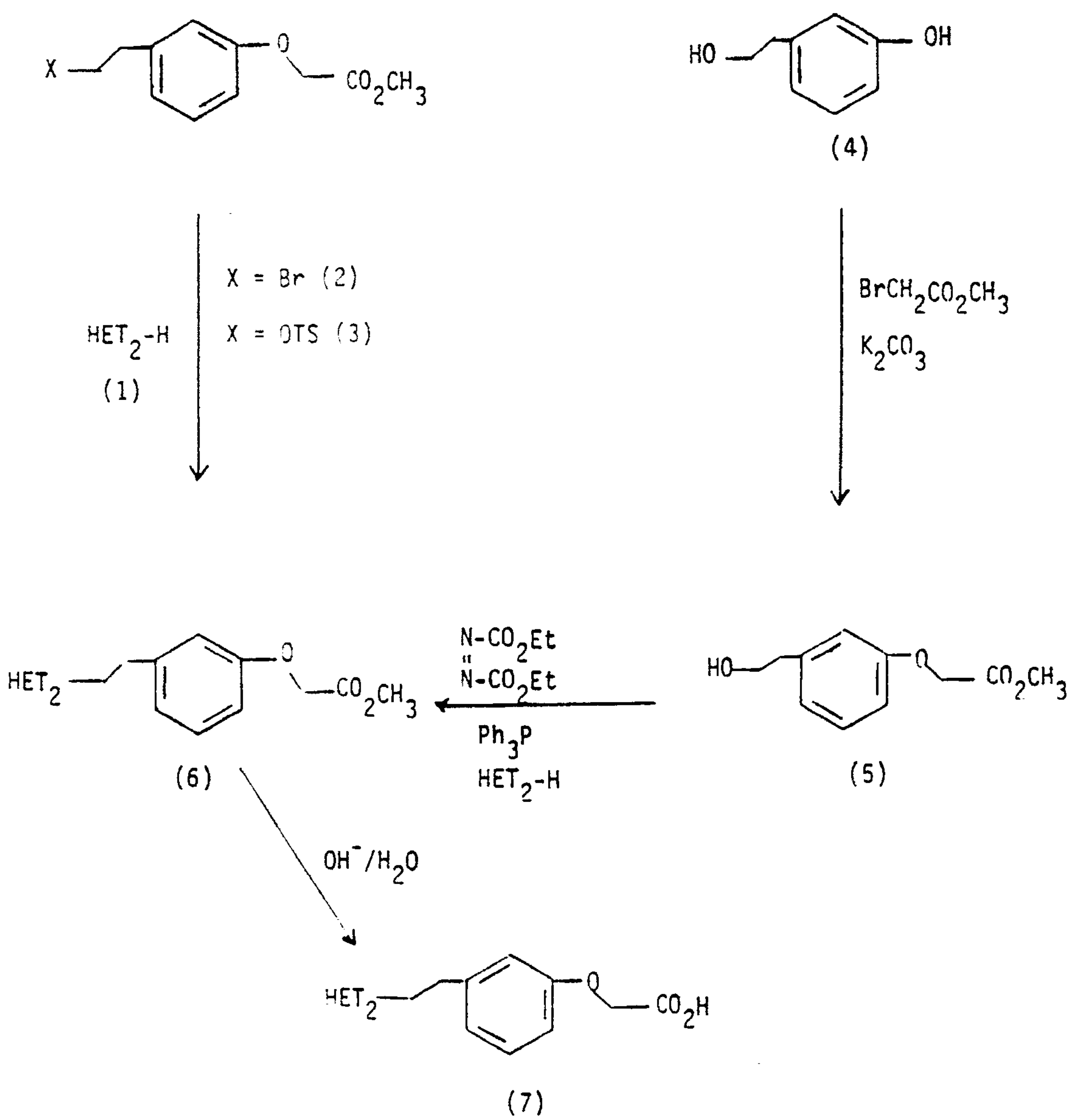
(4)
X = Br (2)
X = OTS (3)
HET2-H
(1)
BrCH2CO2CH3
K2CO3
N-CO2Et
N-CO2Et
Ph3P
HET2-H
(6)
(5)
OH-/H2O
(7)

Scheme 3

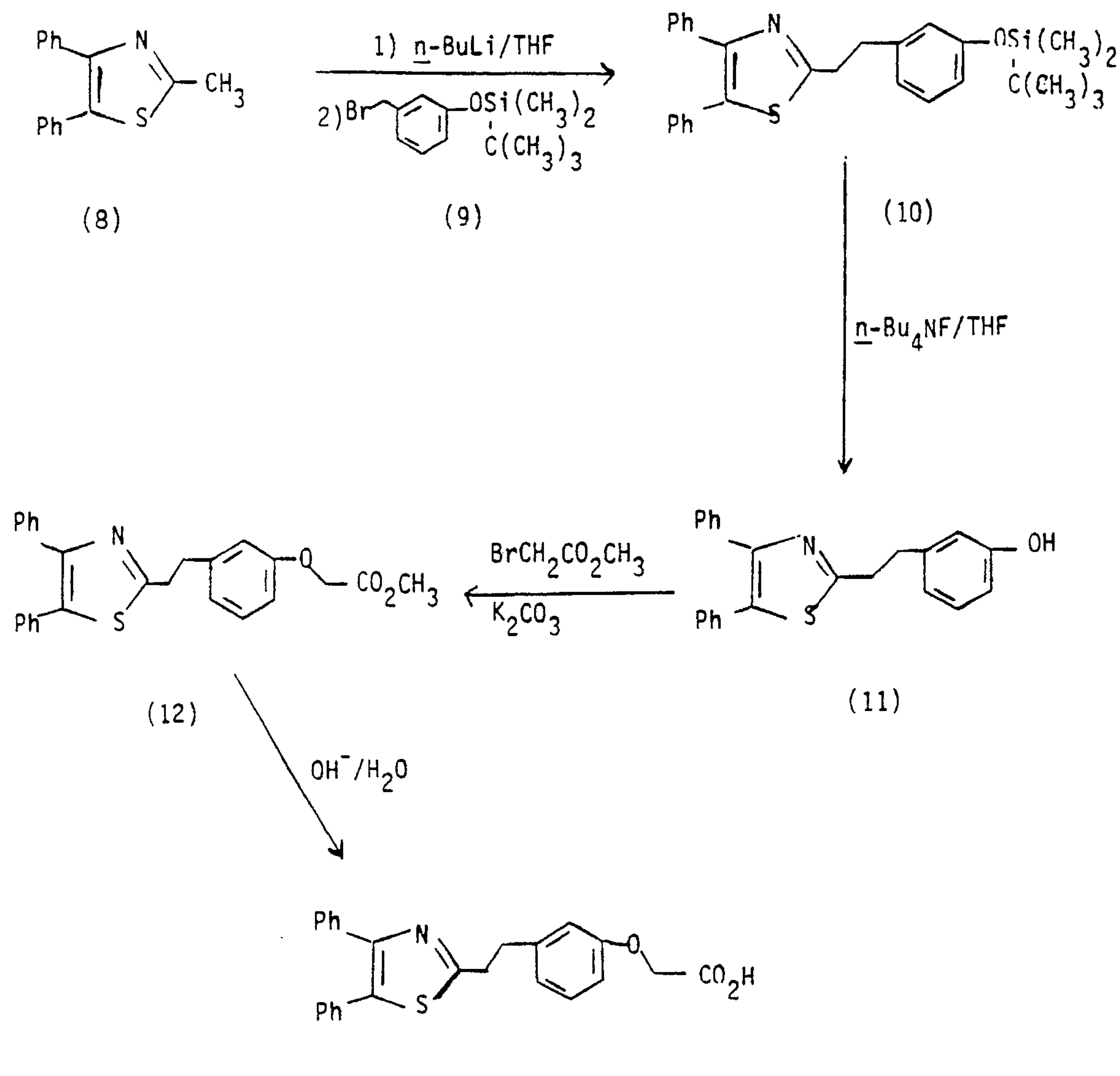


(8) (9) (10)

(12) (11)

(13)

Scheme 4

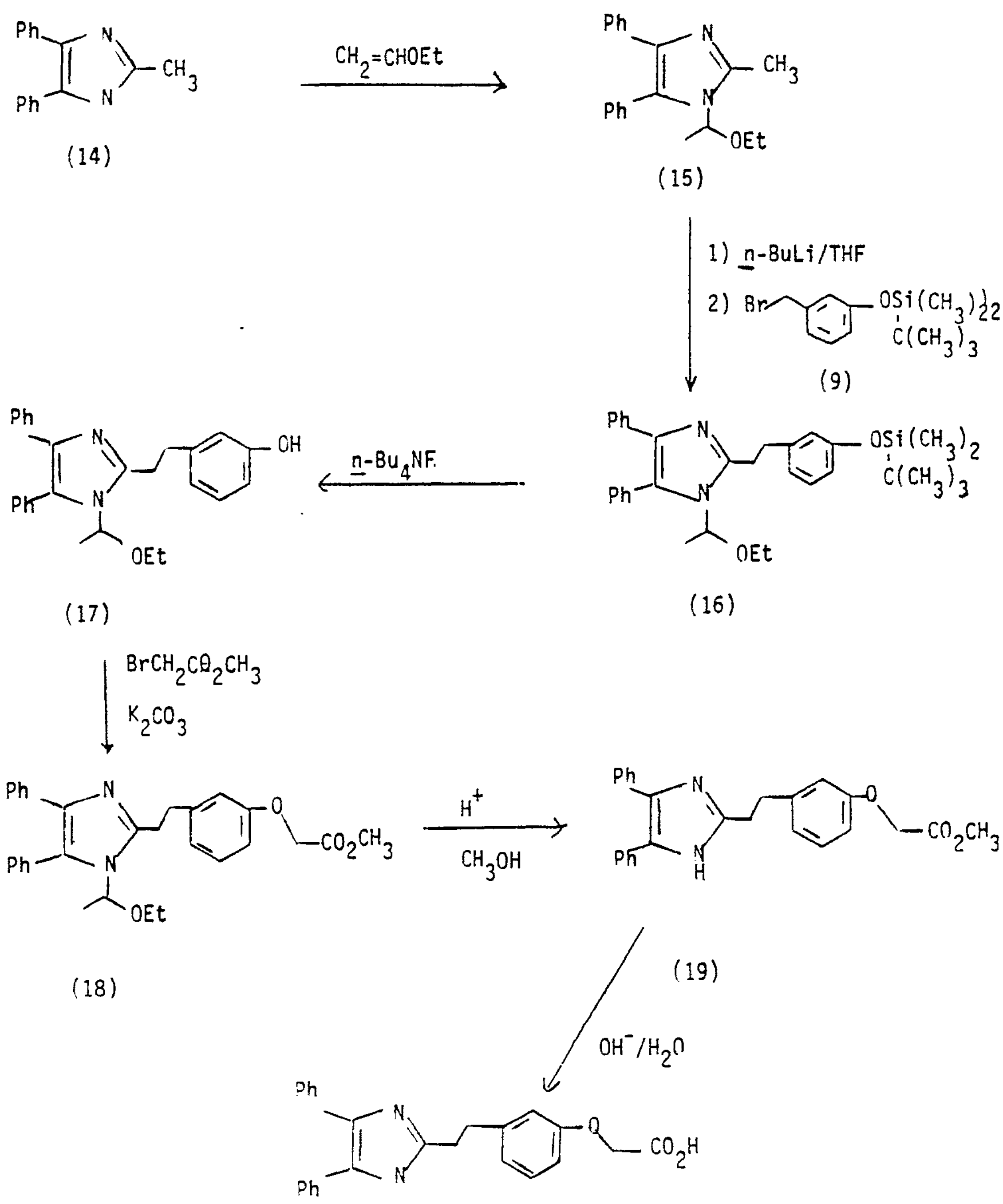


(14) (15) (9) (16) (17) (18) (19) (20)

Scheme 5

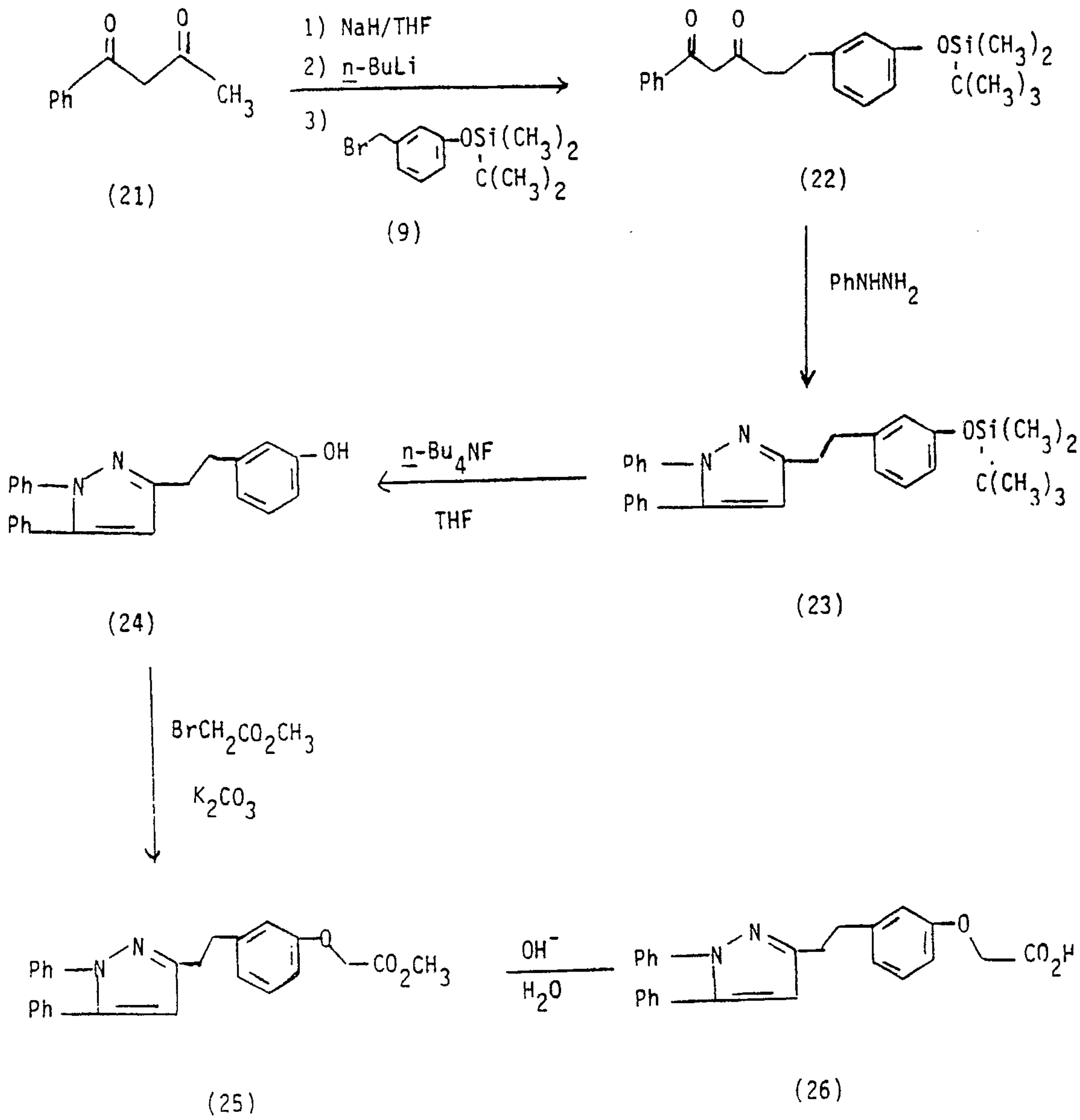


(21) (22) (23) (24) (25) (26)

Scheme 2 depicts several different approaches for synthesis of compounds incorporating the aryloxyacetic acid moiety. Direct alkylation of heterocycle (1) with either bromide (2) or tosylate (TS) (3) available from alcohol (5), was accomplished with sodium hydride in DMF at room temperature or potassium carbonate in DMF at 110° C to furnish esters (6). Alternatively, in some cases, reaction of the heterocycle (1) with alcohol (5) obtained from diol (4) in the presence of diethyl azodicarboxylate and triphenylphosphine provided esters (6) under the mild conditions characteristic of the Mitsunobu reaction, Synthesis, 1-28 (1981). Carboxylic acids (7) were obtained by alkaline hydrolysis of esters (6).

Scheme 3 depicts the synthesis of thiazole derivatives (12) and (13) wherein the aryloxyacetic acid side chain is appended to a carbon. Metallation of 2-methyl-4,5-diphenylthiazole (8) with n-butyllithium followed by quenching with bromide (9) provided the silyl ether (10) which was deprotected with tetra-n-butylammonium fluoride to give phenol (11). Alkylation of phenol (11) with methyl bromoacetate gave ester (12) which was hydrolyzed to the acid (13) under aqueous alkaline conditions.

Scheme 4 depicts preparation of Formula II imidazole derivatives (19) and (20) wherein the aryloxyacetic acid side chain is also appended to a carbon. The synthesis was carried out in a manner analogous

to Scheme 3 but the presence of an active hydrogen in 2-methyl-4,5-diphenylimidazole (14) necessitated introduction of a protecting group onto one of nitrogen atoms prior to metallation. Various protecting groups known to those skilled in the art can be used with the ethoxyethyl moiety described by T.S. Manoharan, et al., J. Org. Chem., 53, 1107-1110 (1988), particularly preferred as it has the advantage of being inexpensive and easily introduced. Heating 2-methyl-4,5-diphenylimidazole (14) with an excess of ethyl vinyl ether in a sealed reaction vessel provided the crystalline N-(1-ethoxyethyl) imidazole (15). Metallation of imidazole (15) with n-butyllithium followed by reaction with bromide (9) provided silyl ether (16). Selective removal of the silyl protecting group was accomplished by exposing (16) to tetra-n-butylammonium fluoride. Alkylation of phenol (17) with methyl bromoacetate afforded ester (18) which was converted to the imidazole (19) by heating in methanol in the presence of concentrated sulfuric acid. Aqueous alkaline hydrolysis gave acid (20).

Scheme 5 depicts preparation of Formula II pyrazoles (25) and (26). Alkylation of the dianion of 1-benzoylacetone (21) with bromide (9) afforded diketone (22). Exposure of (22) to phenylhydrazine provided pyrazole (23). Removal of the silyl protecting group of (23) with tetra-n-butylammonium fluoride and subsequent alkylation of phenol (24) with methyl bromoacetate gave ester (25). Base-induced hydrolysis of ester (25) provided the target acid (26).

Alkali metal salts of Formula II carboxylic acids ($R^1$ is an alkali metal ion) are conventionally prepared by dissolving the acid in a suitable solvent such as methanol, adding a molar equivalent of an alkali base such as sodium methoxide, and precipitating the salt or removing the solvent.

As stated above, the compounds of Formula I and Formula II have pharmacological properties which make them particularly useful as inhibitors of blood platelet aggregation.

Platelet aggregation is considered part of a complex physiological mechanism for formation of a thrombus in the vascular system. Thromboembolic phenomena, i.e., the formation of thrombi, are involved in hemostasis and a number of disease states in mammals including thrombophlebitis, phlebothrombosis, cerebral thrombosis, coronary thrombosis and retinal vessel thrombosis. An increase in propensity for platelet aggregation, sometimes referred to as platelet adhesiveness, is observed following parturition, surgical operations such as coronary artery bypass surgery, organ transplant, angioplasty, prosthetic heart valve implants to name a few and in ischaemic heart disease, artherosclerosis, multiple sclerosis, intracranial tumors, thromboembolism, and hyperlipemia; refer to A. Poplawski, et al., J. Artherosclerosis Research, 8, 721 (1968). Thus, the compounds of the invention which have antithrombogenic actions (inhibit blood platelet aggregation) are useful in prevention or treatment of conditions involving platelet aggregation and thrombosis such as the above. The instant compounds are also considered to have antimetastatic potential in view of their platelet inhibition properties.

The pharmacological properties of the instant compounds can be demonstrated by conventional in vitro and in vivo biological tests such as the following.

## IN VITRO INHIBITION OF HUMAN PLATELET AGGREGATION

The aggregometer method of Born, C.V.R., J. Physiol., (London), 1962, 162, 67-68, as modified by Mustard, J.F., et al., J. Lab. Clin. Med. 1964, 64, 548-599 was used to assess the in vitro activity of the various compounds as to the inhibition of adenosine diphosphate (ADP) and collagen-induced platelet aggregation. The human volunteer donor's arm is cleansed with 70% ethyl alcohol. A sterile 20 ml syringe and needle are used to withdraw 20 ml of blood. The blood is immediately added to a test tube containing 3.8% sodium citrate to prevent clotting (1 part citrate to 9 parts blood).

Platelet rich plasma (PRP) was separated by centrifugation for 10 minutes at 1000 rpm (140xg) from citrated (3.8%) human blood. All glassware used for preparation of PRP is silicon treated. ADP in final concentration of 0.5 mcg/mL or 0.05 mL of a collagen suspension prepared according to the method described by Evans, G., et al., J. Exp. Med., 1968, 128, 877-894 was used to induce aggregation. The various compounds tested were dissolved in dimethylsulfoxide (DMSO) so that 5 mcl added to the platelet rich plasma would yield the desired test concentration. Vehicle control trials were done and compared with aggregation induced in platelet rich plasma containing various concentrations of the test compounds. Dose response curves were obtained and Inhibitor Concentration ($IC_{50}$) values calculated. In this test, the $IC_{50}$ values for dipyridamole, a clinically useful antithrombogenic agent, are 512 mcg/ml vs. ADP and 245 mcg/ml vs collagen. Results for 50% inhibition of ADP-induced aggregation are given in Tables I and II hereinafter for various Formula I and II compounds.

TABLE 1

Inhibition of Human Platelet Aggregation of Formula I Compounds ($IC_{50}$ mcg/ml)

$$HET_1-(CH_2)_nCO_2R$$

| Example | $HET_1$ | n | R | vs. ADP mcg/ml |
|---|---|---|---|---|
| 1 | 3,4-diphenyl-1H-pyrrol-1-yl | 8 | $CH_3$ | 32 |
| 2 | | 8 | H | 4 |
| 3 | 1,6-dihydro-6-oxo-3,4-diphenyl-1-pyridazinyl | 8 | $CH_3$ | 21 |
| 4 | | 8 | H | 2.9 |
| 5 | 1,6-dihydro-6-oxo-3,4-diphenyl-1-pyridazinyl | 7 | $CH_3$ | 7.7 |
| 6 | | 7 | H | 1.6 |
| 7 | 2,3-dihydro-3-oxo-5,6-diphenyl-1,2,4-triazin-2-yl | 8 | $CH_3$ | 6.8 |
| 8 | | 8 | H | 2.2 |
| 9 | 2,3-dihydro-3-oxo-5,6-diphenyl-1,2,4-triazin-2-yl | 7 | $CH_3$ | 4.6 |
| 10 | | 7 | H | 3.1 |
| 11 | 2-oxo-4,5-diphenyl-2H-pyrimidin-1-yl | 8 | $CH_3$ | >32 |
| 12 | | 8 | H | 12 |
| 13 | 2-oxo-4,5-diphenyl-2H-pyrimidin-1-yl | 7 | $CH_3$ | >32 |
| 14 | | 7 | H | 10 |
| 15 | 2,5-dioxo-3,4-diphenyl-imidazolidin-1-yl | 8 | $CH_3$ | >32 |
| 16 | | 8 | H | 11.9 |
| 17 | 5-(diphenylmethyl)-2H-tetrazol-2-yl | 8 | $CH_3$ | >32 |
| 18 | | 8 | H | 4.2 |

TABLE 2

Inhibition of Human Platelet Aggregation of Formula II Compounds ($IC_{50}$ mcg/ml)

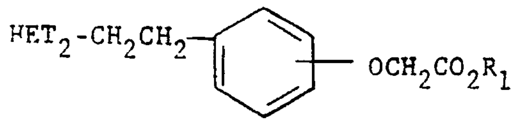

| Example | $HET_2$ | $R_1$ | Vs ADP mcg/ml |
|---|---|---|---|
| 19 | 4,5-diphenyl-1-thiazoyl | $CH_3$ | 0.41 |
| 20 | | H | 0.36 |
| 21 | 4,5-diphenyl-1H-imidazol-2-yl | $CH_3$ | 2.4 |
| 22 | | H | 1.2 |
| 23 | 3,4-diphenyl-1H-pyrazol-1-yl | $CH_3$ | 0.21 |
| 24 | | H | 0.35 |
| 23 | 4,5-diphenyl-1H-pyrazol-1-yl | $CH_3$ | 0.20 |
| 25 | | H | 0.30 |
| 26 | 1,5-diphenyl-1H-pyrazol-3-yl | $CH_3$ | 0.33 |
| 27 | | H | 0.67 |
| 28 | 1,6-dihydro-6-oxo-3,4-diphenyl-1-pyridazinyl | $CH_3$ | 0.75 |
| 29 | | H | 1.0 |
| 30 | 2,3-dihydro-3-oxo-5,6-diphenyl-1,2,4-triazin-2-yl | $CH_3$ | 1.0 |
| 31 | | H | 0.9 |
| 32 | 5-(diphenylmethyl)-2H-tetrazol-2-yl | $CH_3$ | 0.9 |
| 33 | | H | 1.4 |

It is evident that, as a group, the Formula I and II acids are particularly potent inhibitors of ADP-induced aggregation of human platelets. While the esters are generally less active than the parent acid, they are useful as pro-drugs in vivo where they are hydrolyzed to the corresponding acid.

The dosage employed in the therapeutic methods of the instant invention will vary with the form of administration, the particular compound chosen, the subject being tested and the effect desired. Suitable effective doses in animals range from 0.1-50 mg/kg body weight orally and from 0.05-10 mg/kg body weight parenterally (generally characterized as subcutaneous, intramuscular, and intravenous injection). It is contemplated that the effective unit dose in man will range from 1 to 100 mg and preferably from 1 to 20 mg administered one to three times a day. In accordance with conventional clinical practice, the effective dose can be determined by administering a Formula I or II compound at a dosage substantially less than the dose of the compound which is thought to be effective and then increasing the dosage in small increments until the desired effect is achieved.

In carrying out the instant therapeutic methods, the active ingredient of Formula I or II or alkali metal salts of Formula I and II carboxylic acids are preferably administered with a pharmaceutically acceptable carrier and such compositions constitute part of the instant invention. Suitable dosage forms for oral use are tablets, dispersible powders, granules, capsules, syrups and elixirs. Examples of parenteral forms are solutions, suspension, dispersions, emulsions, and the like. The compositions for oral use may contain one or more conventional adjuvants, such as sweetening agents, flavoring agents, coloring agents and preserving agents, in order to provide a composition of suitable pharmaceutical elegance. Tablets may contain the active ingredient in admixture with conventional pharmaceutical acceptable excipients including inert diluents such as calcium carbonate, sodium carbonate, lactose and talc; granulating and disintegrating agents such as starch and alginic acid; binding agents such as starch, gelatin and acacia and lubricating agents such as magnesium stearate, stearic acid and talc. The tablets may be uncoated or coated by known techniques to delay disintegration and absorption in the gastrointestinal tract and thereby provide a sustained action over a longer period. Similarly, suspension, syrups and elixirs may contain the active ingredient in admixture with any of the conventional excipients utilized for the preparation of such compositions such as suspending agents (e.g., methylcellulose, tragacanth, and sodium alginate), wetting agents (e.g., lecithin, polyoxyethylene stearate) and preservatives such as ethyl-p-hydroxybenzoate. Capsules may contain the active ingredient alone or admixed with an inert solid diluent such as calcium carbonate, calcium phosphate and kaolin. The injectible compositions are formulated as known in the art and may contain appropriate dispersing or wetting agents and suspending agents identical or similar to those mentioned above.

The following examples are given by way of illustration and are not to be construed as limiting the invention in any way inasmuch as many variations of the invention are possible within the spirit of the invention.

DESCRIPTION OF SPECIFIC EMBODIMENTS

In the following examples, all temperatures are given in degrees Centigrade. Melting points were recorded on a Thomas-Hoover capillar melting point apparatus and are uncorrected. Proton magnetic resonance ($^1H$-NMR) spectra were recorded on a Bruker AM 300, Bruker WM 360 or Varian Gemini 300 spectrometer. All spectra were determined in $CDCl_3$ or DMSO-$d_6$ unless otherwise indicated and chemical shifts are reported in delta units downfield from the internal standard tetramethylsilane (TMS) and interproton coupling constants are reported in Hertz (Hz). Splitting patterns are designated as follows: s, singlet; d, doublet; t, triplet; q, quartet; m, multiplet; br, broad peak; and dd, doublet of doublet.

EXAMPLE 1

Methyl 3,4-diphenyl-1H-pyrrole-1-nonanoate

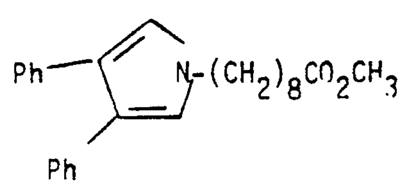

Sodium hydride (789 mg of a 50% dispersion in oil, 16mmol) was washed twice with hexanes and covered with DMF (40mL). 3,4-Diphenylpyrrole (3g, 13mmol), obtained according to K.R. Kopecky, et al., Can. J. Chem., 54, 2645-2651 (1976), was added and the mixture stirred at room temperature until gas evolution ceased. Methyl-9-bromononanoate (3.61g, 14mmol) was added and stirring continued at room temperature. After 15 minutes, the mixture was poured onto 1N hydrochloric acid solution and extracted with diethyl ether (3x). The combined extracts were washed with water (3x), dried over sodium sulfate and concentrated in vacuo to leave an oil which was chromatographed on a column of silica gel. Elution with a mixture of hexanes and diethyl ether (9:1) afforded methyl 3,4-diphenvl-1H-pyrrole-1-nonanoate (3.70g, 69%) as an oil.

Anal. Calcd. for $C_{26}H_{31}NO_2$: C, 80.17; H, 8.02; N, 3.60. Found: C, 79.84; H, 8.02; N, 3.36%.

$^1H$-NMR ($CDCl_3$) delta: 1.20 to 1.50 (8H, m), 1.60 (2H, m), 1.82 (2H, quintet, J=7Hz), 2.29 (2H, t,

J=8Hz), 3.64 (3H, s), 3.87 (2H, t, J=7Hz), 6.74 (2H, s), 7.20 to 7.60 (10H, m).

EXAMPLE 2

3,4-Diphenyl-1H-pyrrole-1-nonanoic Acid

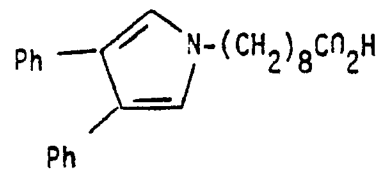

A mixture of methyl 3,4-diphenyl-1H-pyrrole-1-nonanoate (2.50g, 6.4mmol), 5N sodium hydroxide solution (3.86mL, 19mmol) and methanol (50mL) was heated on a steam bath. After 30 minutes, the solvent was evaporated, the residue diluted with 1N HCl solution and extracted with $CH_2Cl_2$. The combined extracts were dried over sodium sulfate, concentrated in vacuo and the residual oil chromatographed on a column of silica gel. Elution with a mixture of diethyl ether and hexanes (3:2) gave 3,4-diphenyl-1H-pyrrole-1-nonanoic acid (2.20g, 91%) as an oil.

Anal. Calcd. for $C_{25}H_{29}NO_2.0.7H_2O$: C, 77.37; H, 7.90; N, 3.61; $H_2O$, 3.25. Found: C, 77.23; H, 7.52; N, 3.42; $H_2O$, 0.43%.

$^1$H-NMR ($CDCl_3$) delta: 1.42 (8H, bs), 1.72 (2H, quintet, J=7Hz), 1.89 (2H, quintet, J=7Hz), 2.42 (2H, t, J=7Hz), 3.92 (2H, t, J=7Hz), 6.83 (2H, s), and 7.20 to 7.40 (10H, m).

EXAMPLE 3

Methyl 6-oxo-3,4-diphenyl-1(6H)-pyridazinenonanoate

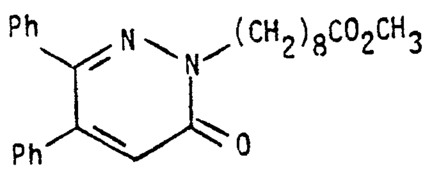

Sodium hydride (840mg of a 60% dispersion in oil, 20mmol) was washed twice with hexanes, covered with DMF (25mL) and 5,6-diphenyl-3(2H)-pyridazinone (4g, 16mmol), obtained according to P. Schmidt, et al., Helvetica Chim. Acta., 37, 134-140 (1954), added. After gas evolution had ceased, the mixture was stirred at room temperature for 30 minutes before adding methyl 9-bromononanoate (4.45g, 18mmol). The mixture was heated briefly to 110°C to give a solution and then allowed to cool before being stirred at room temperature for 1 hour. The mixture was diluted with water and extracted with diethyl ether (3x). The combined extracts were washed with water, dried over sodium sulfate and concentrated to leave an oil. Chromatography on a column of silica gel afforded methyl 6-oxo-3,4-diphenyl-1(6H)-pyridazine nonanoate (6.44g, 95%) as an oil.

Anal. Calcd. for $C_{26}H_{30}N_2O_3$: C, 74.61; H, 7.22; N, 6.69. Found: C, 74.89; H, 7.38; N, 7.23%.

$^1$H-NMR ($CDCl_3$) delta: 1.20 to 1.45 (8H, m), 1.56 (2H, quintet, J=7Hz), 1.85 (2H, quintet, J=7Hz), 2.23 (2H, t, J=7.5Hz), 3.59 (3H, s), 4.20 (2H, t, J=7Hz), 6.88 (1H, s) and 7.00 to 7.40 (10H, m).

EXAMPLE 4

6-Oxo-3,4-diphenyl-1(6H)-pyridazinenonanoic acid

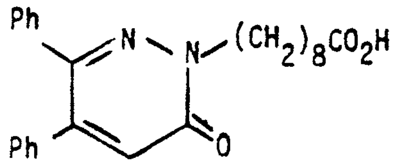

A mixture of methyl 6-oxo-3,4-diphenyl-1(6H)-pyridazinenonanoate (6g, 14mmol), 5N NaOH solution (8.6mL, 43mmol) and methanol (100mL) was heated to reflux on a steam bath. After 30 minutes, the mixture was concentrated, diluted with water and acidified to pH = 1 with 2N HCl solution. The mixture was extracted with $CH_2Cl_2$, the combined extracts dried over sodium sulfate and concentrated to leave an oil that crystallized on standing. Recrystallization from a mixture of $CH_2Cl_2$ and hexanes gave 6-oxo-3,4-diphenyl-1-(6H)-pyridazinenonanoic acid (3.92g, 67%) mp 100-103 °C.

Anal. Calcd. for $C_{25}H_{28}N_2O_3$: C, 74.23; H, 6.98; N, 6.93. Found: C, 74.25; H, 7.13; N, 7.11%.

$^1$H-NMR ($CDCl_3$) delta: 1.20 to 1.50 (8H, m), 1.59 (2H, t), 1.87 (2H, quintet, J = 6.5Hz), 2.29 (2H, t, J = 7Hz), 4.24 (2H, t, J = 7Hz), 6.98 (1H, s), 7.00 to 7.40 (10H, m) and 9.83 (1H, bs).

EXAMPLE 5

Methyl 6-Oxo-3,4-diphenyl-1(6H)pyridazinyloctanoate

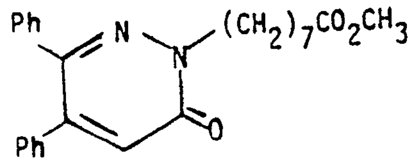

Reaction of 5,6-diphenyl-3(2H)-pyridazone and methyl 8-bromooctanoate according to the procedure of Example 3 provided the title compound as an oil.

Anal. Calcd. for $C_{25}H_{28}N_2O_3$: C, 74.23; H, 6.98; N, 6.93. Found: C, 74.23; H, 7.27; N, 7.34.

EXAMPLE 6

6-Oxo-3,4-diphenyl-1(6H)-pyridazinyloctanoic Acid

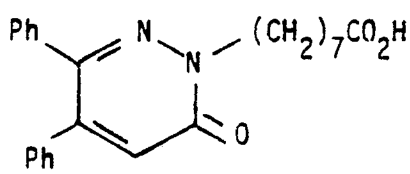

Hydrolysis of methyl 6-oxo-3,4-diphenyl-1(6H)-pyridazinyloctanoate according to the procedure of Example 4 provided the title compound, m.p. 108-111 °C.

Anal. Calcd. for $C_{24}H_{26}N_2O_3.0.2H_2O$: C, 73.15; H, 6.76; N, 7.11; $H_2O$, 0.91. Found: C, 73.20; H, 6.69; N 7.13; $H_2O$, 1.16

EXAMPLE 7

Methyl 3-oxo-5,6-diphenyl-1,2,4-triazine-2(3H)-nonanoate

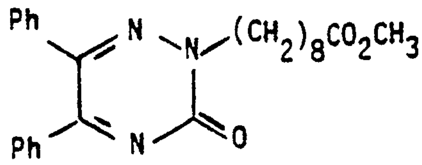

Sodium hydride (577mg of a 60% dispersion in oil, 14mmol) was washed twice with hexanes, covered with DMF (40mL) and 5,6-diphenyl-1,2,4-triazol-3(2H)-one (3g, 12mmol), obtained according to M. Friedman, J. Org. Chem., 30, 859-863 (1965), added. After gas evolution ceased, the mixture was stirred for 15 minutes before adding methyl 9-bromononanoate (3.32g, 13mmol). The mixture was stirred at room temperature for 15 minutes, before being poured onto water and extracted with diethyl ether (3x). The combined extracts were washed with water (3x), dried over sodium sulfate and concentrated to leave an oil which was chromatographed on a column of silica gel. Elution with a mixture of diethyl ether and hexanes (3:2) gave methyl-3-oxo-5,6-diphenyl-1,2,4-triazin-2(3H)-nonanoate (3.09g, 61%).

Anal. Calcd. for $C_{25}H_{29}N_3O_3$: C, 71.58; H, 6.97; N, 10.02. Found: C, 71.59; H, 7.13; N, 10.06%.

$^1$H-NMR ($CDCl_3$) delta: 1.20 to 1.45 (8H, m), 1.58 (2H, quintet, J=6Hz), 1.90 (2H, quintet, J=6Hz), 2.26 (2H, t, J=6Hz), 3.63 (3H, s), 4.21 (2H, t, J=6Hz) and 7.20 to 7.55 (10H, m).

EXAMPLE 8

3-Oxo-5,6-diphenyl-1,2,4-triazine-2(3H)-nonanoic acid

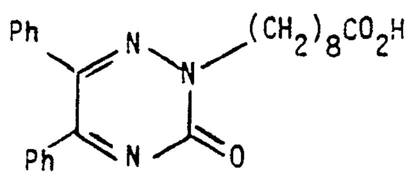

A mixture of methyl-3-oxo-5,6-diphenyl-1,2,4-triazine-2(3H)-nonanoate (1.90g, 4.5mmol), 5N NaOH solution (2.72mL, 13mmol) and methanol (30mL) was heated on a steam bath for 30 minutes. The volatiles were removed in vacuo and the residue diluted with water and dilute HCl solution until pH=1. The mixture was extracted with $CH_2Cl_2$, the combined extracts dried over sodium sulfate and concentrated to give an oil. Chromatography on a column of silica gel using diethyl ether as eluent gave 3-oxo-5,6-diphenyl-1,2,4-triazine-2(1H)-nonanoic acid (1.50g, 81%) which crystallized on standing and had mp 97-100 °C.

Anal. Calcd. for $C_{24}H_{27}N_3O_3$: C, 71.09; H, 6.71; N, 10.36. Found: C, 71.29; H, 6.97; N, 10.21%.

$^1$H-NMR ($CDCl_3$) delta: 1.20 to 1.50 (8H, m), 1.57 (2H, t, J=7Hz), 1.88 (2H, quintet, J=7Hz), 2.27 (2H, t, J=7.5Hz), 4.19 (2H, t, J=7Hz), 7.20 to 7.60 (10H, m) and 10.89 (1H, bs).

EXAMPLE 9

Methyl 3-Oxo-5,6-diphenyl-1,2,4-triazine-2(3H)-octanoate

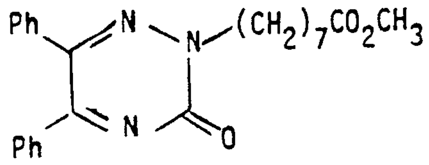

Reaction of 5,6-diphenyl-1,2,4-triazol-3(2H)one and methyl 8-bromooctanoate according to the procedure of Example 7 provided the title compound as an oil

Anal. Calcd. for $C_{24}H_{27}N_3O_3$: C, 71.09; H, 6.71; N, 10.36. Found: C, 71.05; H, 6.87; N, 10.41.

EXAMPLE 10

3-Oxo-5,6-diphenyl-1,2,4-triazin-2(3H)-octanoic Acid

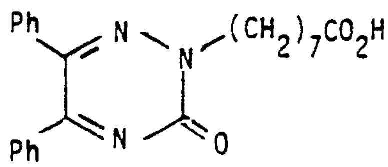

Hydrolysis of methyl 3-oxo-5,6-diphenyl-1,2,4-triazine-2(3H)-octanoate according to the procedure of Example 8 provided the title compound, m.p. 111-113°C.

Anal. Calcd. for $C_{23}H_{25}N_3O_3$: C, 70.57; H, 6.44; N, 10.73. Found: C, 70.35; H, 6.72; N, 10.33.

EXAMPLE 11

Methyl 2-Oxo-4,5-diphenyl-1(2H)-pyrimidinenonanoate

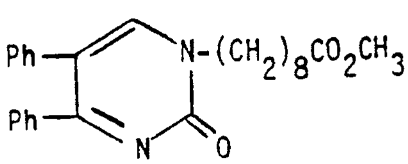

A mixture of 4,5-diphenyl-2(1H)-pyrimidinone (6.00g, 24mmol) obtained according to G.M. Coppola, et al., J. Het. Chem., 16, 545-554 (1979), methyl 9-bromononanoate (6.37g, 25mmol), potassium carbonate (4.00g, 29mmol) potassium iodide (catalytic quantity) and DMF (120mL) was stirred at 110°C under an atmosphere of nitrogen. After 30 minutes, the mixture was cooled, diluted with water and extracted three times with diethyl ether. The combined extracts were washed with water (3x), dried over sodium sulfate and the solvent evaporated. The residual oil was chromatographed on a column of silica gel using diethyl ether as eluent to give methyl 9-[4,5-diphenyl)-2-pyrimidinyl)oxy]nonanoate (3.27g, 32%) as an oil and then with further elution methyl-2-oxo-4,5-diphenyl-1(2H)-pyrimidinenonanoate (6.00g, 59%) as an oil.

Anal. Calcd. for $C_{26}H_{30}N_2O_3$: C, 74.61; H, 7.22; N, 6.69. Found: C, 74.94; H, 7.38; N, 6.89%.

$^1$H-NMR ($CDCl_3$) delta: 1.20 to 1.45 (8H, m), 1.56 (2H, quintet, J=7Hz), 1.81 (2H, quintet, J=7Hz), 2.24 (2H, t, J=7Hz), 3.60 (3H, s), 3.94 (2H, t, J=7Hz), 6.95 to 7.45 (10H, m) and 7.61 (1H, s).

EXAMPLE 12

2-Oxo-4,5-diphenyl-1(2H)-pyrimidinenonanoic Acid

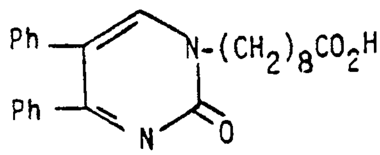

A mixture of methyl 2-oxo-4,5-diphenyl-1(2H)-pyrimidinenonanoate (4.70g, 11mmol), 5N NaOH solution (7.40mL, 36mmol) and methanol (80mL) was stirred at room temperature for 75 minutes and than at reflux for 75 minutes before being cooled and concentrated. The residue was diluted with water and 2N HCl was added until pH=2. The mixture was extracted with $CH_2Cl_2$, the extracts combined, dried over sodium sulfate and the solvent evaporated to leave an oil that crystallized. Recrystallization from a mixture of diethyl ether and $CH_2Cl_2$ afforded 2-oxo-4,5-diphenyl-1(2H)-pyrimidinenonanoic acid (3.35g, 73%), m.p. 120-123 °C.

Anal. Calcd. for $C_{25}H_{28}N_2O_3$: C, 74.23; H, 6.98; N, 6.93. Found: C, 74.22; H, 7.08; N, 6.61%.

$^1$H-NMR ($CDCl_3$) delta: 1.20 to 1.50 (8H, m), 1.56 (2H, bt), 1.80 (2H, bt), 2.27 (2H, t, J=7Hz), 3.94 (2H, t, J=7Hz), 6.95 to 7.55 (10H, m); 7.65 (1H) and 10.56 (1H, bs).

EXAMPLE 13

Methyl 2-Oxo-4,5-diphenyl-1(2H)-pyrimidineoctanoate

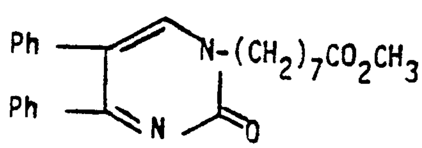

Reaction of 4,5-diphenyl-2(1H)-pyrimidinone and methyl 8-bromooctanoate according to the procedure of Example 11 provided the title compound, m.p. 80-83 °C.

Anal. Calcd. for $C_{25}H_{28}N_2O_3$: C, 74.23; H, 6.98; N, 6.93. Found: C, 74.64; H, 7.21; N, 7.00.

EXAMPLE 14

2-Oxo-4,5-diphenyl-1(2H)pyrimidineoctanoic Acid

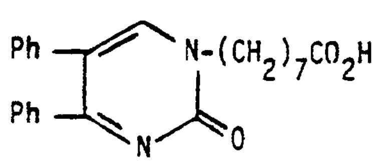

Hydrolysis of methyl 2-oxo-4,5-diphenyl-2(1H)-pyrimidinyloctanoate according to the procedure of Example 12 provided the title compound, m.p. 121-123 °C.

Anal. Calcd. for $C_{24}H_{26}N_2O_3$: C, 73.82; H, 6.71; N, 7.17. Found: C, 73.43; H, 6.83; N, 7.28.

EXAMPLE 15

Methyl 2,5-dioxo-3,4-diphenyl-1-imidazolidinenonanoate

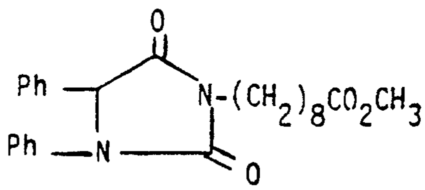

Diethyl azodicarboxylate (1.39g, 8mmol) in THF (5mL, was added dropwise to a stirred solution of methyl 9-hydroxynonanoate (1.50g, 8mmol), 3,4-diphenylimidazolidine-2,5-dione (2.02g, 8mmol) obtained according to the procedure of K.C. Joshi, et al., J. Het. Chem., 18, 1651-1653 (1981) and triphenylphosphine (2.10g, 8mmol) in THF (35mL). The mixture was stirred at room temperature for 72 hours, the THF removed in vacuo and the residue triturated with a mixture of hexanes and diethyl ether. The mixture was filtered, the filtrate concentrated and subjected to chromatography on a column of silica gel using a mixture of hexanes and ethyl acetate (3:2) as eluent. Elution gave methyl 2,5-dioxo-3,4-diphenyl-1-imidazolidinenonanoate (2.78g, 82%), mp 69-72° C.

Anal. Calcd. for $C_{25}H_{30}N_2O_4$: C, 71.07; H, 7.16; N, 6.65. Found: C, 71.13; H, 7.19; N, 6.66%.

$^1$H-NMR ($CDCl_3$) delta: 1.20 to 1.40 (8H, m), 1.50 to 1.70 (4H, m), 2.25 (2H, t, J=6Hz), 3.55 (2H, t, J=6Hz), 3.61 (3H, s), 5.41 (1H, s), 7.03 (1H, t, J=6.5Hz), 7.10 to 7.40 (7H, m) and 7.45 (2H, d, J=6.5Hz).

EXAMPLE 16

2,5-Dioxo-3,4-diphenyl-1-imidazolidinenonanoic acid

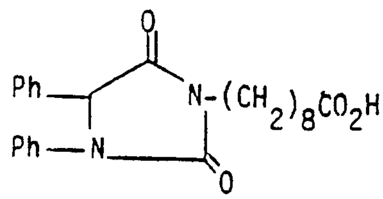

A mixture of methyl 2,5-dioxo-3,4-diphenyl-1-imidazolidinenonanoate (3.00g, 7mmol) and 6N HCl solution (30mL) was heated at reflux for 22 hours. The mixture was cooled, extracted with $CH_2Cl_2$ and the combined extracts washed with water and twice with saturated sodium chloride solution. After drying over sodium sulfate, the solvent was evaporated and the residue chromatographed on a column of silica gel using a mixture of hexanes and ethyl acetate (2:1) as eluent. Elution gave 2,5-dioxo-3,4-diphenyl-1-imidazolidinenonanoic acid (1.06g, 36%) after recrystallization from a mixture of hexanes, diethyl ether and $CH_2Cl_2$ (6:2:2), mp 111.5-112.5° C.

Anal. Calcd. for $C_{24}H_{28}N_2O_4$: C, 70.57; H, 6.91; N, 6.86. Found: C, 70.45; H, 7.05; N, 6.75%.

$^1$H-NMR ($CDCl_3$) delta: 1.20 to 1.40 (6H, m), 1.50 to 1.80 (4H, m), 2.14 (2H, t, J=6Hz), 3.61 (2H, t, J=6Hz), 5.42 (1H, s), 7.09 (1H, t, J=6Hz) and 7.15 to 7.60 (9H, m).

EXAMPLE 17

Methyl 5-(diphenylmethyl)-2H-tetrazole-2-nonanoate

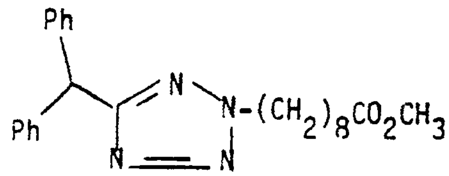

A mixture of 5-(diphenylmethyl)-1H-tetrazole (5g, 21mmol) obtained according to J.W. Cusic, U.S. 3,155,666, methyl 9-bromononanoate (5.84g, 23mmol), potassium carbonate (3.50g, 25mmol), potassium iodide (catalytic quantity) and DMF (75mL) was stirred at 110°C for 15 minutes. The mixture was cooled, diluted with water and extracted three times with diethyl ether. The combined extracts were washed three times with water, dried over sodium sulfate and concentrated in vacuo to give an oil. Chromatography on a column of silica gel using a mixture of hexanes and diethyl ether (1:1) as eluent gave methyl 5-(diphenylmethyl)-2H-tetrazole-2-nonanoate (6.50g, 75%).

Anal. Calcd. for $C_{24}H_{30}N_4O_2$: C, 70.91; H, 7.44; N, 13.78. Found: C, 71.00; H, 7.57; N, 14.24%.

$^1$H-NMR ($CDCl_3$) delta: 1.20 to 1.40 (8H, m), 1.59 (2H, quintet, J=7Hz), 1.96 (2H, quintet, J=7Hz), 2.28 (2H, t, J=7.5Hz), 3.64 (3H, s), 4.53 (2H, t, J=7Hz), 5.81 (1H, s) and 7.10 to 7.50 (10H, m).

EXAMPLE 18

5-(Diphenylmethyl)-2H-tetrazole-1-nonanoic acid

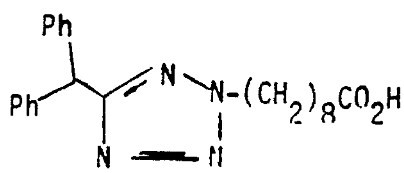

A mixture of methyl 5-(diphenylmethyl)-2H-tetrazole-1-nonanoate (5.00g, 12mmol), 5N NaOH solution (7.40mL, 36mmol) and methanol (100mL) was heated to reflux on a steam bath. After 20 minutes, the mixture was concentrated, diluted with water and 2N HCl solution and a white solid collected by filtration. Recrystallization from a mixture of $CH_2Cl_2$ and hexanes gave 5-(diphenylmethyl)-2H-tetrazole-1-nonanoic acid (4.40g, 90%), mp 68-70°C.

Anal. Calcd. for $C_{23}H_{28}N_4O_2$: C, 70.38; H, 7.19; N, 14.27. Found: C, 70.37; H, 7.24; N, 14.35%.

$^1$H-NMR ($CDCl_3$) delta: 1.28 (8H, bs), 1.58 (2H, quintet, J=6.5Hz), 1.97 (2H, quintet, J=6.5Hz), 2.29 (2H, t, J=7.5Hz), 4.55 (2H, t, J=7Hz), 5.81 (1H, s), 7.15 to 7.40 (10H, m) and 10.52 (1H, bs).

EXAMPLE 19

Methyl (3-[2-(4,5-diphenyl-2-thiazolyl)ethyl]phenoxy]acetate

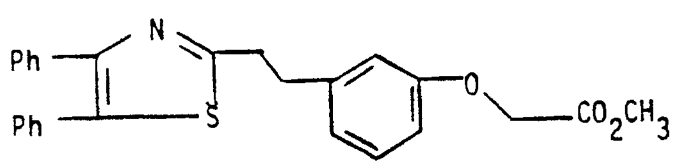

A mixture of 3-[2-(4,5-diphenyl-2-thiazolyl)ethyl]phenol (3.65g, 10mmol), methyl bromoacetate (1.72g, 1.06mL, 11mmol) potassium carbonate (1.69g, 12mmol) and acetonitrile (60mL) was stirred at reflux for 90

minutes. The mixture was filtered and concentrated to leave an oil which was subjected to chromatography on a column of silica gel. Elution with a mixture of hexanes and diethyl ether (2:1) afforded methyl [3-[2-(4,5-diphenyl-2-thiazolyl)ethyl]phenoxy]acetate (3.63g, 82%) as an oil.

Anal. Calcd. for $C_{26}H_{23}NO_3S$: C, 72.70; H, 5.40; N, 3.26. Found: C, 73.13; H, 5.50; N, 3.26%.

$^1$H-NMR ($CDCl_3$) delta: 3.10 to 3.20 (2H, m), 3.30 to 3.40 (2H, m), 3.78 (3H, s), 4.62 (2H, s), 6.76 (1H, dd, J=8Hz, J'=2.5Hz), 6.16 (1H, d, J=2.5Hz), 6.92 (1H, d, J=8Hz), 7.15 to 7.40 (9H, m) and 7.50 to 7.60 (2H, m).

EXAMPLE 20

[3-[2-(4,5-Diphenyl-2-thiazolyl)ethyl]phenoxy]acetic Acid

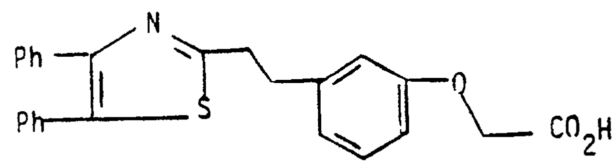

A mixture of methyl [3-[2-(4,5-diphenyl-2-thiazolyl)ethyl]phenoxy]acetate (2.70g, 6mmol), 5N NaOH solution (3.77mL, 18mmol) and methanol (40mL) was heated to reflux on a steam bath. After 20 minutes, the mixture was cooled, the methanol removed and the residue diluted with water and 2N HCl solution. The precipitate was filtered off, washed with water and dried in air. Recrystallization from a mixture of $CH_2Cl_2$ and hexanes provide [3-[2-(4,5-diphenyl-2-thiazolyl)ethyl)phenoxy]acetic acid (1.97g, 75%), mp 118-120 °C.

Anal. Calcd. for $C_{25}H_{21}NO_3S.0.1H_2O$: C, 71.96; H, 5.13; N, 3.36; $H_2O$, 0.43. Found: C, 71.69; H, 5.10; N, 3.06; $H_2O$ 0.30%.

$^1$H-NMR (DMSO-$d^6$) delta: 3.06 (2H, t, J=8Hz), 3.31 (2H, t, J=8Hz), 4.62 (2H, s), 6.73 (1H, d, J=8Hz), 6.90 (2H, bs), 7.19 (1H, t, J=8Hz) and 7.25 to 7.60 (10H, m).

EXAMPLE 21

Methyl [3-[2-(4,5-diphenyl-1H-imidazol-2-yl)ethyl]phenoxy]-acetate

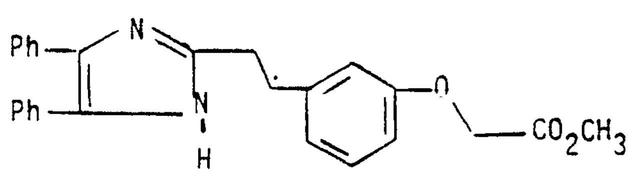

A mixture of 3-[2-[1-(1-ethoxyethyl)-4,5-diphenyl-1H-imidazo-2-yl]ethyl]phenol (6.60g, 16mmol), methyl bromoacetate (2.69g, 1.66mL, 18mmol), potassium carbonate (2.65g, 19mmol), potassium iodide (catalytic amount) and acetonitrile (100mL) was stirred at reflux for 2.5 hours. The mixture was filtered concentrated and the residual oil dissolved in methanol (120mL). Concentrated sulfuric acid (6 drops) was added and the mixture heated to reflux. After 75 minutes, concentrated $H_2SO_4$ (3 drops) was added and the mixture heated at reflux for a further 3.75 hours. The methanol was evaporated, the residue diluted with water and saturated sodium carbonate solution and the mixture extracted with $CH_2Cl_2$. The combined extracts were dried over sodium sulfate and the solvent removed to leave an oil that crystallized on standing for 40 hours. Trituration with diethyl ether gave methyl [3-[2-(4,5-diphenyl-1H-imidazol-2-yl)ethyl]phenoxy]-acetate (5-15g, 78%). An analytical sample was prepared by recrystallizing a sample twice from a mixture of $CH_2Cl_2$, diethyl ether and hexanes and had mp 123-125 °C.

Anal. Calcd. for $C_{26}H_{24}N_2O_3.0.8H_2O$: C, 73.16; H, 6.05; N, 6.57; $H_2O$ 3.38. Found: C, 73.00; H, 5.78; N, 6.56; $H_2O$, 3.92%.

$^1$H-NMR ($CDCl_3$) delta: 2.80 (4H, bs), 3.71 (3H, s), 4.52 (2H, s), 6.50 to 6.70 (3H, m), 6.94 (1H, t, J=7.5Hz), 7.15 to 7.35 (8H, m), 7.40 to 7.45 (2H, m) and 10.13 (1H, bs).

EXAMPLE 22

[3-[2-(4,5-Diphenyl-1H-imidazol-2-yl)ethyl]phenoxyacetic Acid

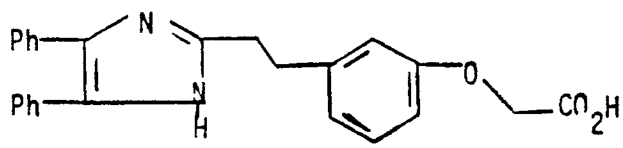

A mixture of methyl [3-(2-(4,5-diphenyl-1H-imidazol-2-yl)ethyl]phenoxy]acetate (1.20g, 3mmol), 5N NaOH solution (1.74mL, 9mmol) and methanol (30mL) was heated to reflux on a steam bath for 20 minutes. The solvent was removed, the residue diluted with water and acidified to pH = 1 with 2N HCl solution. A yellow solid was filtered off and subjected to chromatography on a column of silica gel using a mixture of chloroform and methanol (9:1) as eluent to give [3-[2-(4,5-diphenyl-1H-imidazol-2-yl)ethyl]phenoxy]-acetic acid (0.42g, 36%), mp 244-246 °C.

Anal. Calcd. for $C_{25}H_{22}N_2O_3.H_2O$: C, 72.10; H, 5.81; N, 6.36; $H_2O$ 4.33. Found: C, 72.01; H, 5.72, N, 6.36; $H_2O$, 1.07%.

'H-NMR (DMSO-$d^6$) delta: 3.03 (4H, bs), 4.63 (2H, s), 6.73 (1H, d, J = 8Hz), 6.80 to 6.90 (2H, m), 7.20 (1H, t, J = 8Hz) and 7.25 to 7.60 (10H, m).

EXAMPLE 23

Methyl [3-[2-(3,4-diphenyl-1H-pyrazol-1-yl)ethyl]phenoxy]acetate

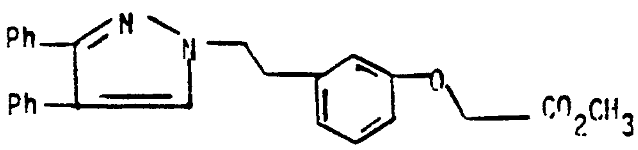

and

Methyl [3-[2-(4,5-diphenyl-1H-pyrazol-1-yl)ethyl]phenoxy]acetate

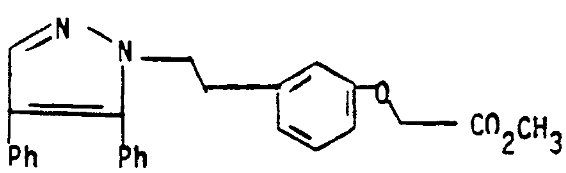

Sodium hydride (0.72g of a 50% dispersion in mineral oil, 15mmol) was washed twice with hexanes, covered with DMF (10mL) and 3,4-diphenyl-1H-pyrazole (2.99g, 13mmol) added. After gas evolution had ceased, the mixture was stirred at 110 °C for 1 hour, cooled to room temperature and methyl [3-[2-[[(4-methylphenyl)sulfonyl]oxy]ethyl]phenoxy]acetate. (4.95g, 12mmol) in DMF (20mL) added. After stirring at room temperature for 15 hours, the mixture was poured onto water and extracted with ethyl acetate. The combined extracts were washed three times with water, once with saturated sodium chloride solution, dried over sodium sulfate and the solvent evaporated. The residual oil was chromatographed repeatedly on silica gel using a mixture of hexane and ethyl acetate (4:1) as eluent. The less polar material was identified as methyl [3-[2-(3,4-diphenyl)-1H-pyrazol-1-yl]ethyl]phenoxy] acetate (1.71g, 30%).

Anal. Calcd. for $C_{26}H_{24}N_2O_3.0.3H_2O$: C, 74.73; H, 5.94; N, 6.71; $H_2O$ 1.29. Found: C, 74.42; H, 6.07; N, 6.55; $H_2O$, 0.65%.

$^1$H-NMR ($CDCl_3$) delta: 3.21 (2H, t, J=7Hz), 3.75 (3H, s), 4.36 (2H, t, J=7Hz), 4.56 (2H, s), 6.60 to 6.80 (3H, m), 7.10 to 7.40 (10H, m) and 7.45 to 7.55 (2H, m).

The more polar material was identified as methyl [3-[2-(4,5-diphenyl-1H-pyrazol-1-yl)ethyl]phenoxy]-acetate (0.66g, 11%).

Anal. Calcd. for $C_{26}H_{24}N_2O_3$: C, 75.71; H, 5.87; N, 6.80. Found: C, 75.84; H, 5.90; N, 6.71%.

$^1$H-NMR ($CDCl_3$) delta: 3.06 (2H, t, J=7Hz), 3.75 (3H, s), 4.16 (2H, t, J=7Hz), 4.48 (2H, s), 6.39 (1H, d, J=1.5Hz), 6.56 (1H, d, J=7.5Hz), 6.72 (1H, dd, J=7.5 Hz, J'=1.5Hz), 6.85 to 7.40 (11H, m) and 7.81 (1H, s).

EXAMPLE 24

[3-[2-(3,4-Diphenyl-1H-pyrazol-1-yl)ethyl]phenoxy]acetic Acid

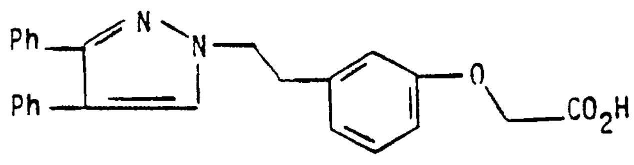

A mixture of methyl [3-[2-(3,4-diphenyl-1H-pyrazol-1-yl)-ethyl]phenoxy]acetate (1.70g, 4mmol), 3N NaOH solution (4.2mL, 12mmol) and methanol (60mL) was heated on a steam bath for 10 minutes. The mixture was concentrated, diluted with water, made pH=1 with dilute HCl solution and extracted with $CH_2Cl_2$. The combined extracts were washed with saturated sodium chloride solution, dried over sodium sulfate and the solvent evaporated. The residue was dissolved in $CH_2Cl_2$ and diluted with hexanes to give [3-[2-(3,4-diphenyl)-1H-pyrazol-1-yl)ethyl]phenoxy]acetic acid (1.27g, 77%), mp 147.5-149°C.

Anal. Calcd. for $C_{25}H_{22}N_2O_3.0.15H_2O$: C, 74.86; H, 5.61; N, 6.98; $H_2O$, 0.67. Found: C, 74.55; H, 5.57; N, 6.96; $H_2O$, 0.43%.

$^1$H-NMR ($CDCl_3$) delta: 3.18 (2H, t, J=7.5Hz), 4.39 (2H, t, J=7.5Hz), 4.57 (2H, s), 6.70 to 6.90 (3H, m), 7.10 to 7.40 (10H, m), 7.45 to 7.50 (2H, m) and 7.60 (1H, bs).

EXAMPLE 25

[3-[2-(4,5-Diphenyl-1H-pyrazol-1-yl)ethyl]phenoxy]acetic Acid

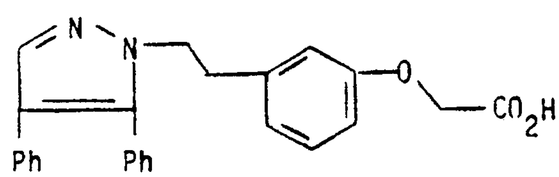

A mixture of methyl [3-[2-(4,5-diphenyl-1H-pyrazol-1-yl)-ethyl)phenoxy]acetate (0.42g, 1mmol), 3N NaOH solution (1.0mL, 3mmol) and methanol (30mL) was heated on a steam bath for 15 minutes before being concentrated in vacuo. The residue was diluted with water, 1N HCl solution added until pH=1 and the mixture extracted with $CH_2Cl_2$. The combined organic phase was washed with saturated sodium chloride solution, dried over sodium sulfate and concentrated in vacuo. The residue was dissolved in $CH_2Cl_2$ and diluted with hexanes to furnish [3-[2-(4,5-diphenyl-1H-pyrazole-1-yl)ethyl]phenoxy]acetic acid (0.28g, 68%) m.p. 133-138°C.

Anal. Calcd. for $C_{25}H_{22}N_2O_3$: C, 75.36; H, 5.57; N, 7.04. Found: C, 75.21; H, 5.48; N, 7.09%.

$^1$H-NMR ($CDCl_3$) delta: 2.99 (2H, t, J=7Hz), 4.21 (2H, t, J=7Hz), 4.57 (2H, s), 6.51 (1H, d, J=7.5Hz), 6.57 (1H, s), 6.78 (1H, dd, J=7.5Hz, J'=2Hz), 6.95 to 7.45 (11H, m) and 7.85 (1H, s).

EXAMPLE 26

Methyl [3-[2-(1,5-diphenyl-1H-pyrazol-3-yl)ethyl]phenoxy]acetate

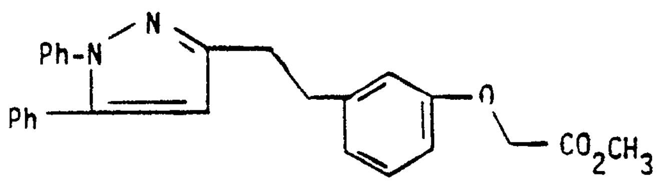

A mixture of 3-[2-(1,5-diphenyl-1H-pyrazol-3-yl)ethyl]phenol (4.50g, 13mmol), methyl bromoacetate (2.23g, 1.375mL, 14.5mmol), potassium carbonate (2.19g, 16mmol), potassium iodide (catalytic amount) and acetonitrile (80mL) was stirred at reflux for 3 hours. The mixture was filtered, concentrated and the residue chromatographed on a column of silica gel prepared in a mixture of hexanes and diethyl ether (9:1). Subsequent elution with a mixture of hexanes and diethyl ether (1:1) furnished methyl [3-[2-(1,5-diphenyl-1H-pyrazol-3-yl)ethyl]phenoxy]acetate (4.83g, 85%) as an oil.

Anal. Calcd. for $C_{26}H_{24}N_2O_3$: C, 75.71; H, 5.86; N, 6.79. Found: C, 75.46; H, 6.13; N, 7.12%.

$^1$H-NMR ($CDCl_3$) delta: 3.04 (4H, s), 3.77 (3H, s), 4.51 (2H, s), 6.30 (1H, s). 6.75 (1H, d, J=7Hz), 6.86 (1H, s), 6.93 (1H, d, J=7Hz), and 7.10 to 7.40 (11H, m).

EXAMPLE 27

[3-[2-(1,5-Diphenyl-1H-pyrazol-3-yl)ethyl]phenoxy]acetic Acid

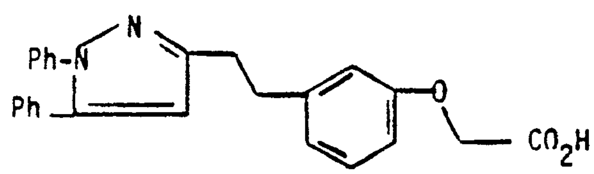

A mixture of methyl [3-[2-(1,5-diphenyl-1H-pyrazol-3-yl)ethyl]phenoxy]acetate (3.50g, 8.5mmol), 5N NaOH solution (5mL, 25mmol) and methanol (70mL) was heated to reflux on a steam bath for 15 minutes. The solvent was evaporated, the residue diluted with water and acidified with 2N HCl to pH=1 A white solid was filtered off and recrystallized from a mixture of hexanes and $CH_2Cl_2$ to give [3-[2-(1,5-diphenyl-1H-pyrazol-3-yl)ethyl]phenoxy]acetic acid (2.85g, 84%), mp 125-127° C.

Anal. Calcd. for $C_{25}H_{22}N_2O_3.0.2H_2O$: C, 74.69; H, 5.62; N, 6.97; $H_2O$, 0.90. Found: C, 74.48; H, 6.08; N, 6.87; $H_2O$, 0.05%.

$^1$H-NMR (DMSO-$d^6$) delta: 2.95 (4H, m), 4.65 (2H, s), 6.51 (1H, s), 6.73 (1H, d, J=7Hz) 6.85 to 6.95 (2H, m), 7.15 to 7.25 (5H, m), 7.25 to 7.40 (6H, m) and 12.65 (1H, bs).

EXAMPLE 28

Methyl [3-[2-(1,6-dihydro-6-oxo-3,4-diphenyl-1-pyridazinyl)ethyl]phenoxy]acetate

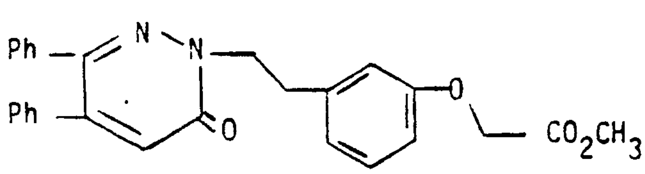

Diethyl azodicarboxylate (2.73g, 2.50mL, 16mmol) was added to a stirred solution of 5,6-diphenyl-3(2H)-pyridazinone (3.00g, 12mmol), methyl [3-(2-hydroxyethyl)phenoxy]acetate (2.80g, 13mmol) and triphenylphosphine (4.12g, 16mmol) in dry THF (75mL) maintained at 0° C. The ice bath was removed and the mixture stirred at room temperature for 1.75 hours before removal of the solvent. The residue was chromatographed twice on columns of silica gel using mixtures of diethyl ether and hexanes (7:3) and then (3:2) as eluent to give methyl [3-[2-(1,6-dihydro-6-oxo-3,4diphenyl-1-pyridazinyl)ethyl]phenoxyacetate (5.12g, 98%), contaminated with 0.6 equivalents of 1,2-dicarbethoxy hydrazine, as a white foam.

Anal. Calcd. for $C_{27}H_{24}N_2O_4.0.3H_2O.0.6C_6H_{12}N_2O_4$: C, 66.64; H, 5.82; N, 8.13; $H_2O$, 0.98. Found: C, 66.45; H, 5.60; N, 7.73; $H_2O$, 0.42%.

$^1$H-NMR ($CDCl_3$) delta: 1.24 (3H, t, J=7Hz), 3.17 (2H, t, J=8Hz), 3.76 (3H, s), 4.17 (2H, q, J=7Hz), 4.49 (2H, t, J=8Hz), 4.59 (1H, s), 6.77 (1H, dd, J=8 Hz, J'=2.5 Hz), 6.83 (1H, d, J=2.5Hz), 6.93(1H, s), 6.93 (1H, m), 7.00 to 7.10 (4H, m) and 7.15 to 7.40 (8H, m).

EXAMPLE 29

[3-[2-(1,6-Dihydro-6-oxo-3,4-diphenyl-1-pyridazinyl)ethyl]phenoxy]acetic Acid

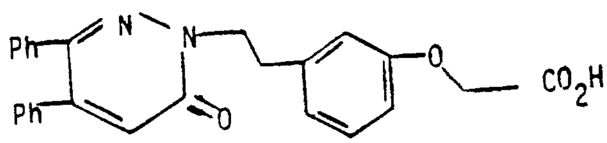

A mixture of methyl [3-[2-(1,6-dihydro-6-oxo-3,4-diphenyl-1-pyridazinyl)ethyl]phenoxy]acetate (4.60g, 11mmol), 5N NaOH solution (3.20mL, 32mmol) and methanol (80mL) was heated to reflux on a steam bath. After 25 minutes, the methanol was evaporated, the residue diluted with water and acidified to pH=1 with 2N HCl solution. A white solid was filtered off and dissolved in $CH_2Cl_2$. Addition of diethyl ether gave [3-[2-(1,6-dihydro-6-oxo-3,4-diphenyl)-1-pyridazinyl)ethyl]phenoxy]acetic acid hydrate dichloromethane solvate (2.60g, 58%), mp 165-167° C.

Anal. Calcd. for $C_{26}H_{22}N_2O_4.0.1H_2O.CH_2Cl_2$: C, 63.20; H, 4.76; N, 5.46; $H_2O$, 0.35. Found: C, 63.41; H, 4.69; N, 5.44; $H_2O$, 0.14%.

$^1$H-NMR (DMSO-d$^6$) delta: 3.06 (2H, t, J=7.5Hz), 4.37 (2H, t, J=7.5Hz), 4.62 (1H, s), 5.74 (2H, s), 6.65 to 6.90 (3H, m), 6.94 (1H, s), 7.00 to 7.45 (11H, m) and 12.96 (1H, bs).

EXAMPLE 30

Methyl [3-[2-(2,3-dihydro-3-oxo-5,6-diphenyl-1,2,4-triazin-2-yl)ethyl]phenoxy]acetate

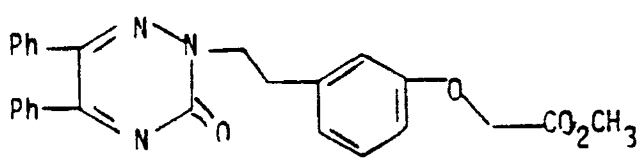

Diethyl azodicarboxylate (2.72g, 2.50mL, 16mmol) was added to a stirred solution of 5,6-diphenyl-1,2,4-triazol-3(2H)-one (3.00g, 12mmol), triphenylphosphine (4.11g, 16mmol) and methyl [3-(2-hydroxyethyl)-phenoxy]acetate (2.78g, 13mmol) in dry THF (75mL) maintained at 0° C under an atmosphere of nitrogen. The ice bath was removed and the mixture stirred at room temperature for 3 hours. The solvent gas evaporated and the residue chromatographed twice on columns of silica gel using a mixture of diethyl ether and hexanes (7:3) as eluent to give methyl [3-[2-(2,3-dihydro-3-oxo-5,6-diphenyl-1,2,4-triazin-2-yl)ethyl]-phenoxy]acetate (6.40g, 90%) as a yellow foam.

Anal. Calcd. for $C_{26}H_{23}N_3O_4.0.5\ H_2O$: C, 69.33; H, 5.38; N, 9.33; $H_2O$, 1.99. Found: C, 69.23; H, 5.19; N, 9.56; $H_2O$, 0.15%.

$^1$H-NMR ($CDCl_3$) delta: 3.19 (2H, t, J=7.5Hz), 3.72 (3H, s), 4.45 (2H, t, J=7.5Hz), 4.56 (1H, s), 6.70 to 6.80 (2H, m), 6.88 (1H, d, J=7.5Hz), 7.10 to 7.50 (11H, m).

EXAMPLE 31

[3-[2-(2,3-Dihydro-3-oxo-5,6-diphenyl-1,2,4-triazin-2-yl)ethyl]phenoxy]acetic Acid

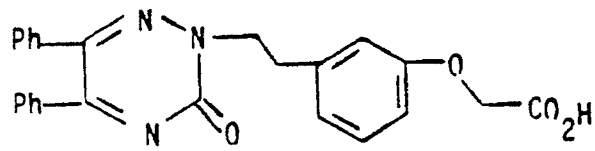

A mixture of methyl [3-[2-(2,3-dihydro-3-oxo-5,6-diphenyl-1,2,4-triazin-2-yl)ethyl]phenoxy]acetate (4.00g, 9mmol), 5N NaOH (3.70mL, 18mmol) and methanol (60mL) was heated to reflux on a steam bath. After 20 minutes, the methanol was evaporated, the residue diluted with water and acidified with 2N HCl solution. A yellow solid was filtered off and recrystallized from a mixture of hexanes and $CH_2Cl_2$ to give [3-[2-(2,3-dihydro-3-oxo-5,6-(diphenyl-1,2,4-triazin-2-yl)ethyl]phenoxy]acetic acid (2.79g, 72%), mp 179-182° C.

Anal. Calcd. for $C_{25}H_{21}N_3O_4.0.5H_2O$: C, 68.80; H, 5.09; N, 9.63; $H_2O$, 2.06. Found: C, 68.81; H, 4.79; N, 8.64; $H_2O$, 0.51%.

$^1$H-NMR (DMSO-d$^6$) delta: 3.10 (2H, t, J=7.5Hz), 4.36 (2H, t, J=7.5 Hz), 4.62 (2H, s), 6.70 to 6.95 (3H, m), 7.10 to 7.60 (11H, m) and 12.97 (1H, bs).

EXAMPLE 32

Methyl [3-[2-[5-(diphenylmethyl)-2H-tetrazol-2-yl]ethyl]-phenoxy]acetate

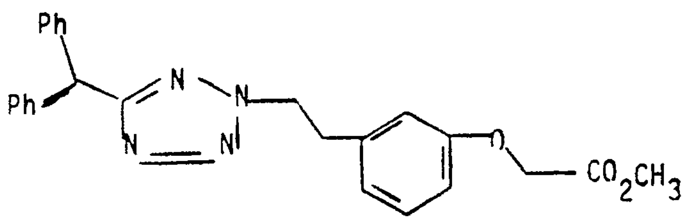

Diethyl azodicarboxylate (0.96g, 5.5mmol) was added to a stirred solution of 5-(diphenylmethyl)-2H-tetrazole (1.00g, 4.2mmol), methyl [3-(2-hydroxyethyl)-phenoxy]acetate (0.98g, 4.6mmol) and triphenylphosphine (1.44g, 5.5mmol) in dry THF (17mL) cooled to 0° C in an ice bath. The ice bath was removed and the mixture stirred at room temperature for 1 hour before the solvent was evaporated. The residue was chromatographed on a column of silica gel using a mixture of hexanes and ethyl acetate (3:1) to give methyl [3-[2-[5-(diphenylmethyl)-2H-tetrazol-2-yl]ethyl]phenoxy]acetate (1.06g, 58%) as a colorless oil.

Anal. Calcd. for $C_{25}H_{24}N_4O_3$: C, 70.08; H, 5.65; N, 13.08. Found: C, 70.03; H, 5.73; N, 12.66%.

$^1$H-NMR ($CDCl_3$) delta: 3.26 (2H, t, J=7.5Hz), 3.78 (3H, s), 4.53 (2H, s), 4.79 (2H, t, J=7.5Hz), 5.77 (1H, s), 6.65 to 6.80 (3H, m) and 7.10 to 7.40 (11H, m).

EXAMPLE 33

[3-[2-[5-(Diphenylmethyl)-2H-tetrazol-2-yl]ethyl]phenoxy]-acetic Acid

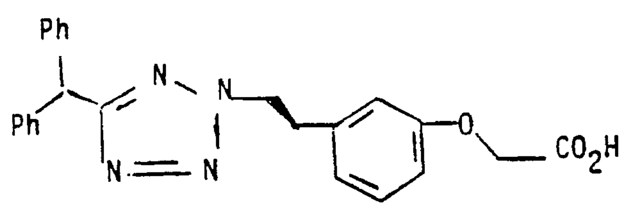

A mixture of methyl [3-[2-[5-(diphenylmethyl)-2H-tetrazol-2-yl]ethyl]phenoxy]acetate (0.84g, 2mmol), 3N NaOH solution (2.00mL, 6mmol) and methanol (35mL) was heated for 10 minutes on a steam bath. The methanol was evaporated, the residue diluted with water and acidified to pH = 1 with 1N HCl solution. The mixture was extracted three times with $CH_2Cl_2$, the combined extracts washed with saturated sodium chloride solution and dried over sodium sulfate. Evaporation of the solvent left an oil which was chromatographed on a column of silica gel using a mixture of chloroform and methanol (93:7) as eluent to give [3-[2-[5-(diphenylmethyl)-2H-tetrazol-2-yl]-ethyl]phenoxy]acetic acid (0.50g, 61%) as a colorless oil.

Anal. Calcd. for $C_{24}H_{22}N_4O_3.0.2H_2O$: C, 68.96; H, 5.41; N, 13.41; $H_2O$, 0.86. Found: C, 68.58; H, 5.42; N, 13.05; $H_2O$, 0.54%.

$^1$H-NMR ($CDCl_3$) delta: 3.25 (2H, t, J = 7.5Hz), 4.54 (2H, s), 4.78 (2H, t, J = 7.5Hz), 5.80 (1H, s), 6.60 to 6.85 (3H, m), 7,20 to 7.40 (11H, m) and 9.62 (1H, bs).

EXAMPLE 34

Preparation of Intermediates

(34-1)

3-[2-(4,5-Diphenyl-2-thiazolyl)ethyl]phenol

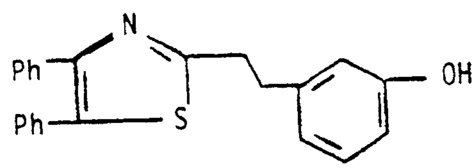

n-Butyllithium (1.53g, 24mmol) in hexane (9.5mL) was added dropwise to a stirred solution of 2-methyl-4,5-diphenylthiazole (5g, 20mmol) in dry THF (150mL) maintained at -78° C under an atmosphere of nitrogen. After 25 minutes a solution of [3-(bromomethyl)phenoxy]dimethyl(1,1-dimethylethyl)silane (6.60g, 22mmol) in THF (10mL) was added dropwise, the mixture stirred at -78° C for 10 minutes and the allowed to warm to room temperature. The reaction mixture was poured onto saturated ammonium chloride solution, extracted with $CH_2Cl_2$, the extracts combined, dried over sodium sulfate and concentrated in vacuo to leave an oil which was dissolved in THF (25mL). A solution of tetra-n-butylammonium fluoride (6.77g, 26mmol) in THF (25.86mL) was added and the mixture stirred at room temperature for 30 minutes before being diluted with 1N HCl solution (100mL). The mixture was extracted with $CH_2Cl_2$, the combined extracts dried over sodium sulfate and concentrated to leave an oil. Chromatography on a column of silica gel using a mixture of hexanes and diethyl ether (2:1) as eluent furnished 3-[2-(4,5-diphenyl-2-thiazolyl)ethyl]phenol (4.13g, 58%), mp 166-168° C after recrystallization from a mixture of hexanes and $CH_2Cl_2$.

Anal. Calcd. for $C_{23}H_{19}NOS$: C, 77.28; H; 5.36; N, 3.92. Found: C, 77.22; H, 5.46; N, 3.71%.

$^1$N-NMR ($CDCl_3$) delta: 3.04 to 3.14 (2H, m), 3.20 to 3.40 (2H, m), 5.89 (1H, s), 6.60 to 6.75 (2H, m) 6.80 to 6.90 (1H, m), 7.13 (1H, t, J = 8Hz), 7.20 to 7.45 (8H, m) and 7.50 to 8.00 (2H, m).

(34-2)

1-(1-Ethoxyethyl)-2-methyl-4,5-diphenyl-1H-imidazole

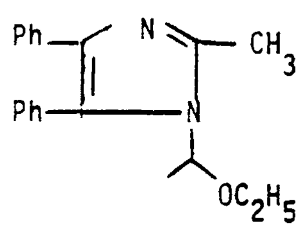

A mixture of 2-methyl-4,5-diphenylimidazole (3.00g, 13mmol), ethyl vinyl ether (9.23g, 12.30mL,

130mmol) and hydroquinone (0.14g, 1.2mmol) was heated at 180°C in a sealed vessel for 17 hours. The excess ethyl vinyl ether was evaporated and the residue chromatographed on a column of silica gel using a mixture of diethyl ether and hexanes (3:2) as eluent. Elution gave 1-(1-ethoxyethyl)-2-methyl-4,5-diphenyl-1H-imidazole (2.68g, 68%), mp 87-89°C.

Anal. Calcd. for $C_{20}H_{22}N_2O$: C, 78.40; H, 7.24; N, 9.14. Found: C, 78.64; H, 7.31; N, 9.56%.

$^1$H-NMR ($CDCl_3$) delta: 1.09 (3H, t, J=7Hz), 1.55 (3H, d, J=6Hz), 2.62 (3H, s), 3.20 and 3.35 (2H, m), 5.07 (1H, q, J=6Hz) and 7.00 to 7.50 (10H, m).

(34-3)

3-[2-[1-(1-Ethoxyethyl)-4,5-diphenyl-1H-imidazol-2-yl]ethyl]-phenol

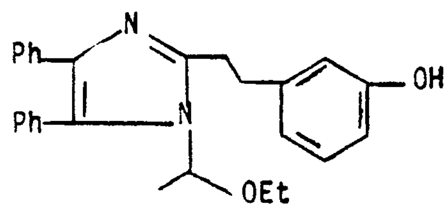

n-Butyllithium (3.89g, 61mmol) in hexane (24.31mL) was added dropwise to a stirred solution of 1-(1-ethoxyethyl)-2-methyl-4,5-diphenyl-1H-imidazole (15.50g, 51mmol) in dry THF (300mL) maintained at -78°C under an atmosphere of nitrogen. The mixture was stirred for 45 minutes and [3-(bromomethyl)-phenoxy]-dimethyl(1,1-dimethylethyl)silane (16.77g, 50mmol) added dropwise. After 10 minutes, the mixture was poured onto water, extracted with $CH_2Cl_2$, the extracts combined and dried over sodium sulfate. Evaporation of the solvent left an oil which was dissolved in THF (75mL) and a solution of tetra-n-butylammonium fluoride (17.22g, 66mol) in THF (65mL) added. The solution was stirred at room temperature for 30 minutes, the solvent evaporated and the residue diluted with water. After addition of 2N HCl solution (about 20mL) to pH=5, the mixture was extracted with $CH_2Cl_2$. The combined extracts were dried over sodium sulfate, concentrated in vacuo and the residual material chromatographed on a column of silica gel. Elution with a mixture of diethyl ether and hexanes (1:1) gave 3-[2-[1-(1-ethoxyethyl)-4,5-diphenyl-1H-imidazol-2-yl]ethyl]-phenol (8.18g, 39%), mp 165.5-167°C after recrystallization from a mixture of $CH_2Cl_2$ and hexanes.

Anal. Calcd. for $C_{27}H_{28}N_2O_2.0.4H_2O$: C, 77.27; H, 6.92; N, 6.68; $H_2O$, 1.72. Found: C, 77.31; H, 6.93; N, 6.29; $H_2O$, 1.75%.

$^1$H-NMR ($CDCl_3$) delta: 0.99 (3H, t, J=7Hz), 1.45 (3H, d, J=6Hz), 3.00 to 3.40 (6H, m), 5.05 (1H, q, J=6Hz), 6.60 (1H, dd, J=7Hz, J'=1.5Hz), 6.74 (2H, m), 7.00 to 7.30 (4H, m), 7.30 to 7.45 (4H m), 7.45 to 7.60 (3H, m) and 9.30 (1H, s).

(34-4)

5-[3-[Dimethyl(1,1-dimethylethyl)siloxy]phenyl]-1-phenyl-1,3-pentanedione

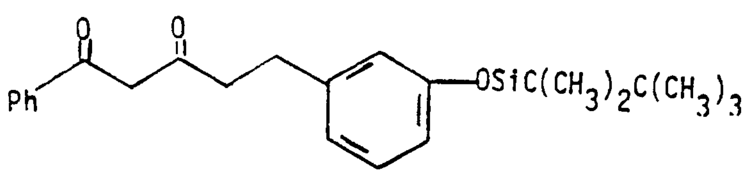

Sodium hydride (2.13g of a 50% dispersion in mineral oil, 44mmol) was washed twice with hexanes, covered with dry THF (150mL) and 1-benzoylacetone (6.00g, 37mmol) added portionwise. The mixture was stirred at room temperature under an atmosphere of nitrogen for 10 minutes to give a slurry which was cooled to -20°C. n-Butyllithium (2.60g, 40mmol) in hexane (16.3mL) was added dropwise to provide a solution which was stirred for 20 minutes prior to the dropwise addition of [3-(bromomethyl)phenoxy]-dimethyl(1,1-dimethylethyl)silane (11.17g, 37mmol). After 15 minutes, the mixture was poured onto saturated ammonium chloride solution and extracted with $CH_2Cl_2$. The combined extracts were dried over sodium sulfate, concentrated in vacuo and the residual oil chromatographed on a column of silica gel. Elution with a mixture of hexanes and diethyl ether (9:1) cave 5-[3-[dimethyl(1,1-dimethylethyl)siloxy]-phenyl]-1-phenyl-1,3-pentanedione (9.00g, 63%).

(34-5)
3-[2-(1,5-Diphenyl-1H-pyrazol-3-yl)ethyl]phenol

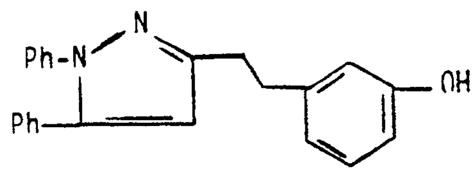

A mixture of 5-[3-[dimethyl(1,1-dimethylethyl)siloxy]phenyl]-1-phenyl-1-3-pentanedione (8 80g, 23mmol), phenylhydrazine (2.73g, 2.50mL, 25mL), pyridine (3mL) and methanol (70mL) was stirred at room temperature. After 20 hours, phenylhydrazine (0.3mL) was added and stirring continued for an additional 7 hours. The solvent was evaporated and the residue chromatographed on a column of silica gel using a mixture of hexanes and diethyl ether (9:1) as eluent to give 3-[2-[3-(dimethyl-(1,1-dimethylethyl)-siloxy]-phenyl]-1-(diphenylmethyl)-5-phenyl-1H-pyrazole (10.27g, 98%) of which (9.20g, 20mmol) was dissolved in THF (180mL). A solution of tetra n-butylammonium fluoride (6.89g, 26mmol) in THF (26.34mL) was added and the mixture stirred at room temperature for 30 minutes. The solvent was evaporated, the residue diluted with 1N HCl solution and extracted with $CH_2Cl_2$. The combined extracts were dried over sodium sulfate and concentrated to give a white solid which was triturated with diethyl ether and filtered to give 3-[2-(1,5-diphenyl-1H-pyrazol-3-yl)ethyl]phenol (5.31g, 77%). An analytical sample recrystallized from ethanol had mp 211-212 °C.

Anal. Calcd. for $C_{23}H_{20}N_2O$: C, 81.15; 5.92; N, 8.23. Found: C, 80.90; H, 6.24; N, 8.22%.

$^1$H-NMR (DMSO-$d^6$) delta: 2.87 (4H, bs), 3.35 (1H, s), 6.49 (1H, s), 6.58 (1H, dd, J=7Hz, J'=1.5Hz), 6.67 to 6.70 (2H, m), 7.07 (1H, t, J=7Hz), 7.10 to 7.30 (4H, m), and 7.30 to 7.50 (6H, m).

The compounds of the instant invention wherein $HET_2$ is the heterocyclic radical 4,5-diphenyl-1H-imidazol-2-ylare further characterized by Formula VIII

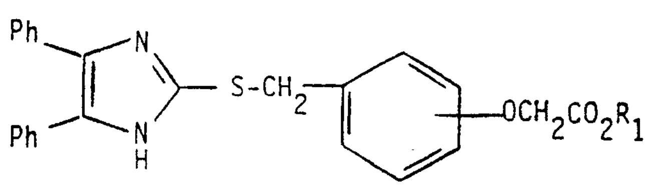

(VIII)

wherein $R_1$ is hydrogen, lower alkyl or an alkali metal ion and the radical $-OCH_2CO_2R$ is attached in the 3 or 4 ring position.

Formula VIII compounds are obtained by a process comprising:

(a) hydrolyzing a compound of Formula IX

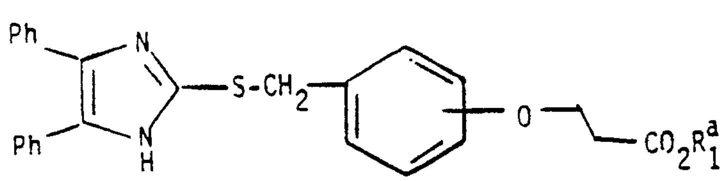

(IX)

wherein $R_1^a$ is lower alkyl; or

(b) esterifying a compound of Formula X

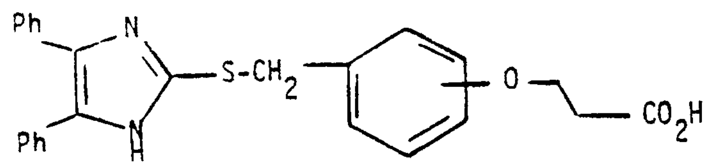

(X)

or;

(c) alkylating 4,5-diphenyl-2-imidazolethiol with a compound of Formula XI

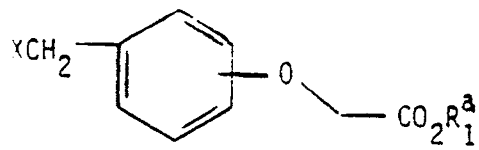

·XI)

wherein $R_1^a$ is lower alkyl and X is halogen or an aryl sulfonate.

The compounds of Formula VIII have pharmacological properties similar to those of Formula I and II compounds. In vitro inhibition of human platelet aggregation test results for the compounds of Examples 35 and 36 are given hereinafter.

TABLE 3

Inhibition of Human Platelet Aggregation of Formula VIII Compounds ($IC_{50}$ mcg/ml)

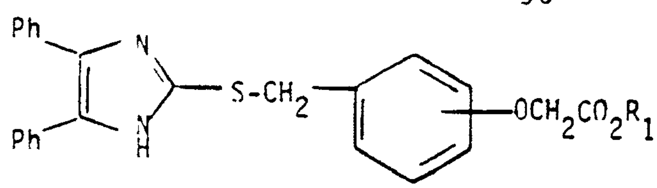

| Example | $R_1$ | VsADP mcg/ml |
|---|---|---|
| 35 | $CH_3$ | 0.42 |
| 36 | H | 0.41 |

EXAMPLE 35

Methyl [3-[[(4,5-diphenyl-1H-imidazol-2-yl)thio]methyl]phenoxy]acetate

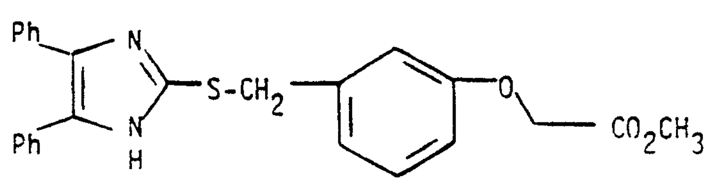

Sodium hydride (684 mg of a 50% dispersion, 14 mmol) was washed three times with hexane and

covered with DMF (60 mL). 4,5-Diphenyl-1-imidazolethiol (3g, 12 mmol) was added to afford a yellow solution after hydrogen evolution had ceased. Methyl (3-chloromethylphenoxy)acetate (2.68 g, 12.5 mmol) was added and the mixture stirred at room temperature for 1.5 hours and then at 100° C for 0.5 hour. After cooling, the mixture was diluted with water and extracted with diethyl ether (3 times). The combined extracts were washed with water (3 times), dried and concentrated in vacuo. Crystallization of the residual solid from a mixture of hexanes and $CH_2Cl_2$ afforded methyl [3-[[4,5-diphenyl-1H-imidazol-2-yl)thio]methyl]phenoxy]-acetate (3.00 g, 58%), m.p. indistinct.

Anal. Calcd. for $C_{25}H_{22}N_2O_3S$: C, 69.75; H, 5.15; N, 6.51. Found: C, 69.79; H, 5.23; N, 6.24.

1H-NMR ($CDCl_3$) delta: 3.72 (3H, s), 4.13 (2H, s), 4.56 (2H, s), 6.75 to 6.80 (3H, m) and 7.10 to 7.50 (12H, m).

EXAMPLE 36

## [3-[[(4,5-Diphenyl-1H-imidazol-2-yl)thio]methyl]phenoxy]acetic Acid

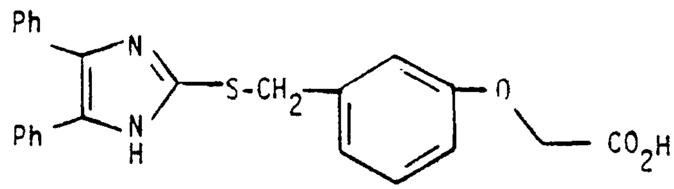

A mixture of methyl [3-[[(4,5-diphenyl-1H-imidazol-2-yl)thio]methyl]phenoxy]acetate (2.00 g, 4.6 mmol), lithium hydroxide monohydrate (586 mg, 14 mmol), methanol (35 mL) and water (10 mL) was stirred at room temperature for 2 hours. The solvent was evaporated, the residue diluted with water and 2N HCl solution added to pH=7. A white solid was filtered off and recrystallized from aqueous DMF to give [3-[[(4,5-diphenyl-1H-imidazol-2-yl)thio]methyl]phenoxy]acetic acid (1.33 g, 69%), m.p. 233-235° C.

Anal. Calcd. for $C_{24}H_{20}N_2O_3S$: C, 69.21; H, 4.84; N, 6.73. Found: C, 69.43; H, 4.92; N, 6.92.

1H-NMR (DMSO-$D^6$) delta: 4.34 (2H, s), 4.60 (2H, s), 6.77 (1H, dd, J=8Hz, J'=2Hz), 6.90 to 7.00 (2H, m), 7.05 to 7.60 (12H) and 12.40 to 13.00 (1H, bs).

## Claims

**1.** A compound of Formula I

$HET_1$-$(CH_2)_nCO_2R$ (I)

wherein

| | |
|---|---|
| n | is 6 to 9; |
| R | is hydrogen or lower alkyl or an alkali metal ion ; and |
| $HET_1$ | is a heterocyclic radical selected from the group consisting of |

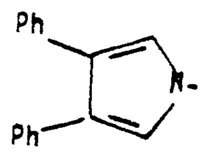

(a) 3,4-diphenyl-1H-pyrrol-1-yl,

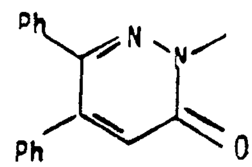

(b) 1,6-dihydro-6-oxo-3,4-diphenyl-1-pyridazinyl,

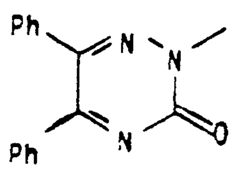

(c) 2,3-dihydro-3-oxo-5,6-diphenyl-1,2,4-triazin-2-yl,

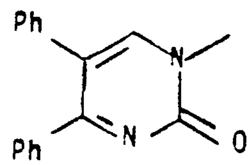

(d) 1-oxo-4,5-diphenyl-2H-pyrimidin-1-yl,

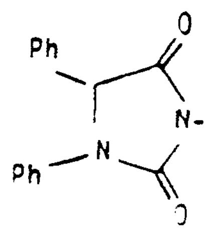

(e) 2,5-dioxo-3,4-diphenylimidazolidin-1-yl,

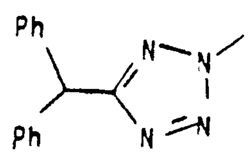

(f) 5-(diphenylmethyl)-2H-tetrazol-2-yl,

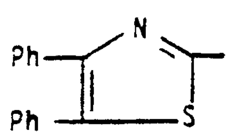

(g) 4,5-diphenyl-2-thiazoyl,

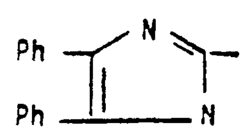

(h) 4,5-diphenyl-1H-imidazol-2-yl, and

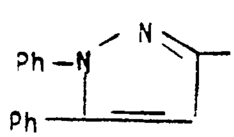

(i) 1,5-diphenyl-1H-pyrazol-3-yl .

**2.** A compound of Formula II

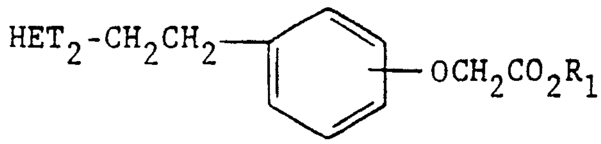

(II)

wherein

$R_1$ is hydrogen, lower alkyl or an alkali metal ion, and the radical $-OCH_2CO_2R_1$ is attached in the 3 or 4 ring position;

$HET_2$ is a heterocyclic radical selected from the group consisting of

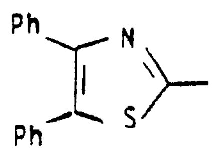

(a) 4,5-diphenyl-2-thiazoyl,

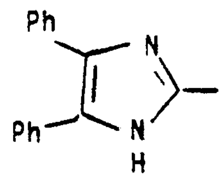

(b) 4,5-diphenyl-1H-imidazol-2-yl,

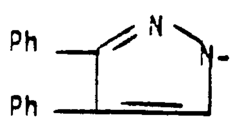

(c) 3,4-diphenyl-1H-pyrazol-1-yl,

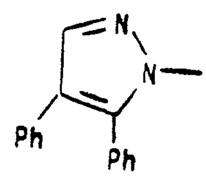

(d) 4,5-diphenyl-1H-pyrazol-1-yl,

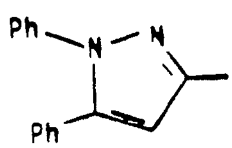

(e) 1,5-diphenyl-1H-pyrazol-3-yl,

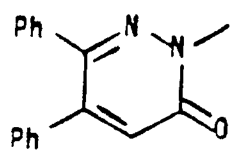

(f) 1,6-dihydro-6-oxo-3,4-diphenyl-1-pyridazinyl,

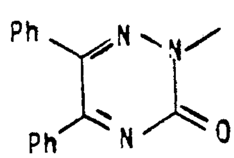

(g) 2,3-dihydro-3-oxo-5,6-diphenyl-1,2,4-triazin-2-yl, and

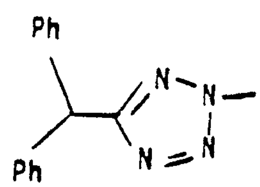

(h) 5-(diphenylmethyl)-2H-tetrazol-2-yl.

3. A compound of Formula VIII

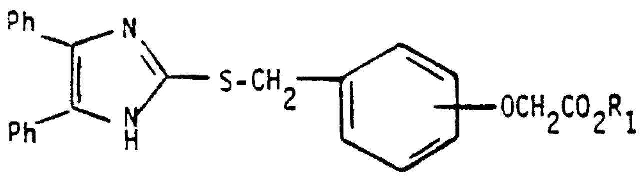

(VIII)

wherein $R_1$ is hydrogen, lower alkyl or an alkali metal ion, and the radical $-OCH_2CO_2R_1$ is attached in the 3 or 4 ring position.

4. The compounds according to claim I which are methyl 3,4-diphenyl-1H-pyrrole-1-nonanoate;

3,4-diphenyl-1H-pyrrole-1-nonanoic acid;

methyl 6-oxo-3,4-diphenyl-1(6H) pyridazinenonanoate;

oxo-3,4diphenyl-1(6H)-pyridazinenonanoic acid;

methyl 6-oxo-3,4-diphenyl-1(6H)-pyridazinyloctanoate;

6-oxo-3,4-diphenyl-1(6H)-pyridazinyl-octanoic acid;

methyl 3-oxo-5,6-diphenyl-1,2,4-triazine-2(3H)-nonanoate;

3-oxo-5,6-diphenyl-1,2,4-triazine-2(3H)-nonanoic acid;

methyl 3-oxo-5,6-diphenyl-1,2,4-triazine-2(3H)-octanoate ;

3-oxo-5,6-diphenyl-1,2,4-triazine-2(3H)-octanoic acid ;

methyl 2-oxo-4,5-diphenyl-1(2H)-pyrimidinenonanoate ;

2-oxo-4,5-diphenyl-1(2H)-pyrimidinenonanoic acid ;

methyl 2-oxo-4,5-diphenyl-1(2H)-pyrimidineoctanoate ;

2-oxo-4,5-diphenyl-1(2H)pyrimidineoctanoic acid ;

methyl 2,5-dioxo-3,4-diphenyl-1-imidazolidinenonanoate;

2,5-dioxo-3,4-diphenyl-1-imidazolidinenonanoic acid;

methyl 5-(diphenylmethyl)-2H-tetrazole-2-nonanoate; and

5-(diphenylmethyl)-2H-tetrazole-1-nonanoic acid.

5. The compounds according to claim 2 which are methyl (3-[2-(4,5-diphenyl-2-thiazolyl)ethyl]phenoxy]-acetate;

[3-[2-(4,5-diphenyl-2-thiazolyl)ethyl]phenoxy]acetic acid;

methyl [3-[2-(4,5-diphenyl-1H-imidazol-2-yl)ethyl]phenoxy]acetate;

[3-[2-(4,5-diphenyl-1H-imidazol-2-yl)ethyl]phenoxyacetic acid;

methyl [3-[2-(3,4-diphenyl-1H-pyrazol-1-yl)ethyl]phenoxy]acetate;

methyl [3-[2-(4,5-diphenyl-1H-pyrazol-1-yl)ethyl]phenoxy]acetate ;

[3-[2-(3,4-diphenyl-1H-pyrazol-1-yl)ethyl]phenoxy]acetic acid ;

[3-[2-(4,5-diphenyl-1H-pyrazol-1-yl)ethyl]phenoxy]acetic acid ;

methyl [3-[2-(1,5-diphenyl-1H-pyrazol-3-yl)ethyl]phenoxy]acetate ;

[3-[2-(1,5-diphenyl-1H-pyrazol-3-yl)ethyl]phenoxy]acetic acid ;

methyl [3-[2-(1,6-dihydro-6-oxo-3,4-diphenyl-1-pyridazinyl)ethyl]phenoxy]acetate ;

[3-[2-(1,6-dihydro-6-oxo-3,4-diphenyl-1-pyridazinyl)ethyl]phenoxy]acetic acid ;

methyl [3-[2-(2,3-dihydro-3-oxo-5,6-diphenyl-1,2,4-triazin-2-yl)ethyl]phenoxy]acetate;

[3-[2-(2,3-dihydro-3-oxo-5,6-diphenyl-1,2,4-triazin-2-yl)ethyl]phenoxy]acetic acid;

methyl [3-[2-[5-(diphenylmethyl)-2H-tetrazol-2-yl]ethyl]-phenoxy]acetate ; and

[3-[2-[5-(diphenylmethyl)-2H-tetrazol-2-yl]ethyl]phenoxy]-acetic acid .

6. The compounds according to claim 3 which are methyl [3-[[(4,5-diphenyl-1H-imidazol-2-yl)thio]methyl]-phenoxy]acetate ; and

[3-[[(4,5-diphenyl-1H-imidazol-2-yl)thio]methyl]phenoxy]acetic acid.

7. A process for preparing the compounds of Formula I as defined in claims 1 and 4 comprising:
(a) hydrolyzing a compound of Formula ($I^a$)

$HET_1$-$(CH_2)_nCO_2R^a$ ($I^a$)

wherein $HET_1$ is as defined above, n is 6-9 and $R^a$ is lower alkyl, or
(b) esterifying a compound of Formula ($I^b$)

$HET_1$-$(CH_2)_nCO_2H$ ($I^b$)

wherein $HET_1$ is as defined above and n is 6-9 with a lower alkanol, or
(c) alkylating $HET_1$-H wherein $HET^1$ is as defined above with a compound of Formula (III)

X-$(CH_2)_nCO_2R^a$ (III)

wherein X is halogen, preferably bromine,, n is 6 to 9, and $R^a$ is lower alkyl.

8. A process for preparing the compounds of formula II as defined in claims 2 and 5 comprising
(a) hydrolyzing a compound of Formula $II^a$

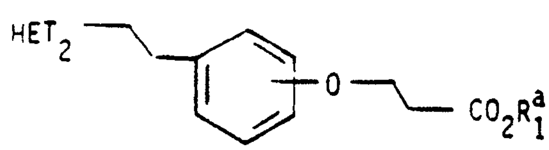

($II^a$)

wherein $Het_2$ is as defined above and $R_1^a$ is lower alkyl; or
(b) esterifying a compound of Formula $II^b$

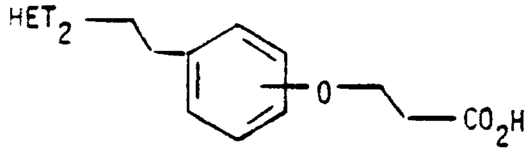

(II$^b$)

wherein $HET_2$ is as defined above; or

(c) alkylating $HET_2$-H wherein $HET_2$ is as defined above with a compound of Formula (V)

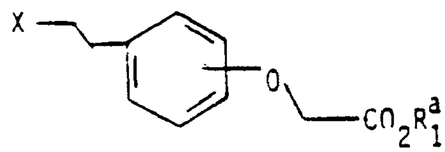

(V)

wherein $R_1^a$ is lower alkyl and X is halogen or an aryl sulfonate; or

(d) alkylating a compound of Formula (VI)

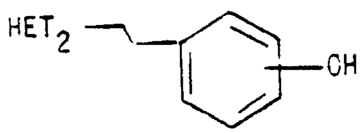

(VI)

wherein $HET_2$ is as defined above with $BrCH_2CO_2R_1^a$ wherein $R_1^a$ is lower alkyl; or

(e) hydrolyzing a compound of Formula VII

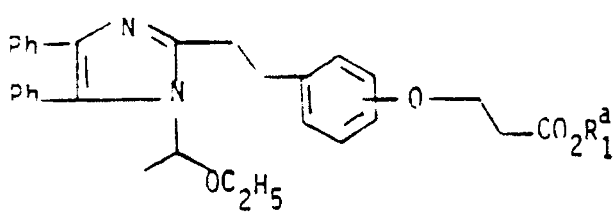

(VII)

9. A process for preparing the compound of Formula VIII as defined in claims 3 and 6, comprising:

(a) hydrolyzing a compound of Formula IX

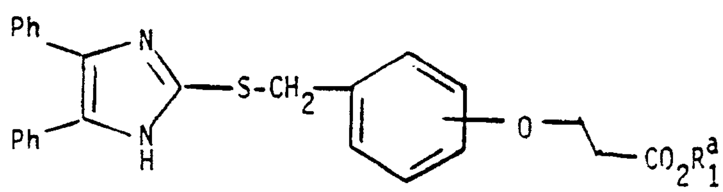

(IX)

wherein $R_1^a$ is lower alkyl; or

(b) esterifying a compound of Formula X

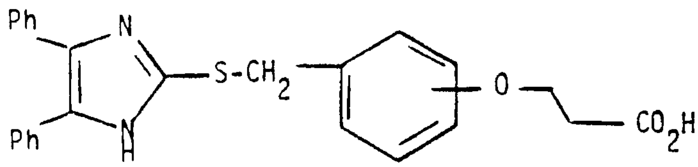

(X)

or;

(c) alkylating 4,5-diphenyl-2-imidazolethiol with a compound, of Formula XI

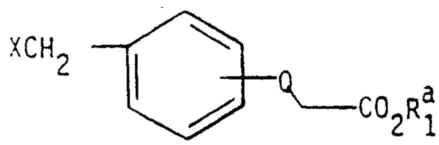

(XI)

wherein $R_1^a$ is lower alkyl and X is halogen or an aryl sulfonate.

**10.** A pharmaceutical composition ccmprising at least one compound according to anyone of claims 1 to 6 and a pharmaceutically acceptable carrier.

**11.** A process for preparing the pharmaceutical composition of claim 10 which comprises incorporating at least one compound according to anyone of claims 1 to 6 into a pharmaceutically acceptable carrier.

**12.** The use of at least one compound according to anyone of claims 1 to 6 for preparing a pharmaceutical composition for inhibiting blood platelet aggregation.